(19) Europäisches Patentamt
European Patent Office
Office européen des brevets

(11) EP 3 641 735 B1

(12) **EUROPEAN PATENT SPECIFICATION**

(45) Date of publication and mention of the grant of the patent:
**24.02.2021 Bulletin 2021/08**

(21) Application number: **18827035.9**

(22) Date of filing: **18.12.2018**

(51) Int Cl.:
***A61K 9/20*** *(2006.01)* ***A61K 31/454*** *(2006.01)*
***A61P 17/04*** *(2006.01)*

(86) International application number:
**PCT/EP2018/085448**

(87) International publication number:
**WO 2019/097091 (23.05.2019 Gazette 2019/21)**

(54) **PHARMACEUTICAL TABLET COMPOSITION COMPRISING BILASTINE FORM 3 AND A WATER-SOLUBLE FILLER**

PHARMAZEUTISCHE TABLETTENZUSAMMENSETZUNG MIT BILASTINFORM 3 UND WASSERLÖSLICHEM FÜLLSTOFF

COMPOSITION DE COMPRIMÉ PHARMACEUTIQUE COMPRENANT UNE FORME DE BILASTINE 3 ET UNE CHARGE SOLUBLE DANS L'EAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.12.2017 IN 201711045443**
**30.01.2018 EP 18154203**
**02.11.2018 IN 201811041577**

(43) Date of publication of application:
**29.04.2020 Bulletin 2020/18**

(73) Proprietor: **Alfred E. Tiefenbacher (GmbH & Co. KG)**
**22767 Hamburg (DE)**

(72) Inventors:
- **GUJJAR, Chaitanya Yogananda**
  **Telangana 502 319 (IN)**
- **UPPALA, Susheel Prakash**
  **Telangana 502 319 (IN)**
- **DONGA, Nani Prasad**
  **Telangana 502 319 (IN)**
- **BANDLA, Srimannarayana**
  **Telangana 502 319 (IN)**
- **RALLABANDI, Bala Ramesha Chary**
  **Telangana 502 319 (IN)**
- **STAVER, Ruslan**
  **22607 Hamburg (DE)**
- **SCHLEHAHN, Hendrik**
  **23843 Travenbrueck (DE)**

(74) Representative: **Hertin und Partner**
**Rechts- und Patentanwälte PartG mbB**
**Kurfürstendamm 54/55**
**10707 Berlin (DE)**

(56) References cited:
**EP-A1- 1 505 066** **WO-A1-2017/017301**
**CN-A- 106 692 090**

- **"The Speciality Excipient Neusilin", , 1 July 2009 (2009-07-01), pages 1-24, XP055250935, Retrieved from the Internet: URL:http://www.harke.com/fileadmin/images/pharma/Broschueren/Fuji_Neusilin.pdf [retrieved on 2016-02-17]**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 641 735 B1

## Description

The invention relates to a pharmaceutical composition in the form of a tablet, comprising a) a crystalline form of bilastine according to crystalline (polymorph) form 3, wherein the crystalline form has characteristic peaks at 6.47, 12.81, 15.70, and 17.71 ± 0.2 degrees 2-theta in a powder X-ray diffraction pattern, b) a water-soluble filler, and optionally c) a water-insoluble filler. The invention further relates to a method of preparing a pharmaceutical composition in the form of a tablet comprising dry granulation of a blend of the tablet components and compression of the granules to a tablet, or direct compression of a blend to a tablet. The invention relates further to the medical use of the pharmaceutical composition in the treatment of allergic rhino-conjunctivitis and/or urticaria.

## BACKGROUND OF THE INVENTION

Bilastine (INN) is known under the chemical name 2-[4-(2-(4-(1-(2-ethoxyethyl)-1H-benzimidazol-2-yl)piperidin-1-yl)ethyl)phenyl]-2-methylpropionic acid and CAS number 202189-78-4. It has the following chemical structure:

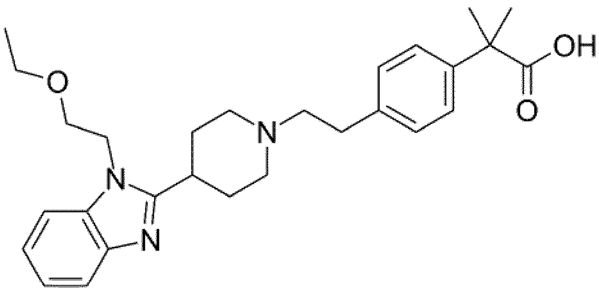

Bilastine is a non-sedating, long-acting histamine antagonist with selective peripheral $H_1$ receptor antagonist affinity and no affinity for muscarinic receptors. Its effectiveness is similar to cetirizine, fexofenadine and desloratadine. Bilastine can be classified into the same chemical group as many of the new antihistamines on the market, although it is not structurally derived, nor is it a metabolite or enantiomer of any of them, but an original molecule designed with the intent of fulfilling all the requirements of a second-generation antihistamine.

It was developed by FAES Farma (former name: Fabrica Espanola de Productos Quimicos y Farmaceuticos SA). Bilastine is approved in the EU for 20 mg once daily dosing for the relief of symptoms of allergic rhino conjunctivitis and urticaria in adults and adolescents. Bilastine is marketed by FAES and its European licensing partner Menarini under different tradenames, e.g. as Bilaska in France and as Bitosen in Germany. Bilastine was first disclosed in EP0818454.

EP1505066 discloses a polymorphic form of bilastine, described as polymorphic form 1. According to EP1505066, bilastine can exist in three different polymorphic forms, called polymorph 1, polymorph 2 and polymorph 3. The procedure described in EP818454 generates a mixture of polymorphs 2 and 3. Polymorph 2, polymorph 3, and their mixture are subsequently converted into polymorph 1 by the procedures of EP1505066, as shown in examples 1-5.

The public assessment report (PAR) of BfArM for 20 mg bilastine tablets of FAES discloses similar information, namely that three polymorphic forms have been identified during development of bilastine by FAES and the active substance consists of polymorphic form 1. Furthermore, the PAR discloses that FAES manufactures tablets by using direct compression of bilastine with the following excipients: microcrystalline cellulose, sodium carboxymethyl starch (also known as sodium starch glycolate), anhydrous colloidal silica and magnesium stearate.

WO 2014/026657 (Zentiva) discloses the preparation of polymorphic forms 1 and 2 of bilastine and discloses not only IR data, but also XRD patterns. No formulations or particular pharmaceutical compositions are disclosed.

SK 7066 Y1 (Zentiva) discloses the preparation of novel hydrate forms of bilastine named dihydrate form A and dihydrate form B and their XRD data. No formulations or particular pharmaceutical compositions are disclosed.

WO 2017/017301 (Urquima) discloses the preparation of some hydrate forms of bilastine, for example forms Alpha and Eta, as well as the preparation of polymorphic forms 1, 2, and 3 and their XRD, IR, and DSC data. According to WO 2017/017301, the neat polymorphic forms 1 and 2 of bilastine and hydrates Alpha and Eta are stable on storage, whilst form 3 is converted partially into the form 1 leading to a mixture of form 3 with form 1 after 1 month storage at 25°C/60%RH (ICH long term conditions) and at 40°C/75%RH (ICH accelerated conditions), and the phase transformation is increasing further after 2 months under accelerated conditions to afford a mixture of form 1 with form 3. Example 10 of WO 2017/017301 discloses pharmaceutical formulations using the hydrate forms Alpha and Eta and with neat form 2 (polymorphic form 2). The formulations contain the same excipients as the reference product, as described in the PAR, mentioned above. No formulations of form 3 are disclosed. The XRD pattern of hydrate form Alpha in WO 2017/017301

is essentially the same as the XRD pattern of dihydrate form A, disclosed in SK 7066 Y1 (Zentiva), leading to the conclusion that "hydrate Alpha" is the same form as "dihydrate form A", only with a different name.

WO2017/167949 (KRKA) discloses novel hydrate forms K1 and K2 of bilastine. No formulations or particular pharmaceutical compositions are disclosed. The same forms K1 and K2 are disclosed in the IP.com disclosure IPCOM000247653D, together with a further neat form called "K3", characterized by XRD, FT-IR, and DSC data. The XRD pattern of form K3 in the figure 11 of IPCOM000247653D is essentially the same as the XRD pattern of form 3, disclosed in figure 7 of WO 2017/017301 (Urquima), leading to the conclusion that "form K3" is the same form as "form 3", only with a different name. No formulations or particular pharmaceutical compositions are disclosed.

CN106692090A (VENTUREPHARM AVENTIS PHARMA) discloses bilastine tablets and methods for their production based on wet granulation. The use of magnesium aluminum silicate is mentioned as a lubricant. No information on preferred polymorph forms of bilastine is disclosed.

Despite the provision of various polymorph forms of bilastine and pharmaceutical compositions comprising the same, further developments are required for improved or more efficient means of formulating bilastine using a simplified and reliable method to provide stable formulations for medical administration.

## SUMMARY OF THE INVENTION

In light of the prior art the technical problem underlying the invention was the provision of improved or alternative means for pharmaceutical compositions comprising bilastine that do not exhibit the disadvantages of the prior art. A further objective of the invention was the provision of simplified means for formulating bilastine, preferably simplified means for formulating the polymorph form 3 of bilastine, in a stable composition.

This problem is solved by the features of the independent claims. Preferred embodiments of the present invention are provided by the dependent claims.

The invention therefore relates to a pharmaceutical composition in the form of a tablet, comprising a) a crystalline form 3 of bilastine, wherein the crystalline form 3 has characteristic peaks at 6.47, 12.81, 15.70, and 17.71 $\pm$ 0.2 degrees 2-theta in a powder X-ray diffraction pattern, b) a water-soluble filler, and optionally c) a water-insoluble filler.

In one embodiment of the invention, the pharmaceutical composition is characterized in that said composition is prepared by dry granulation of a blend of the components a), b) and optionally c) and compression of the granules to a tablet. In a preferred embodiment, the method is characterized by blending the components a), b) and optionally c), roller compaction and granulation of the blend, milling the granules, lubricating the granules and subsequent compression of the granules to a tablet.

The dry granulation of the blend represents an established, cost-efficient, reliable and high-throughput approach towards formulation, which represents an improvement in efficiency and reliability over alternative methods of production, for example in comparison to wet granulation followed by a subsequent tabletting. In one embodiment, the dry granulation of the present invention further represents an inherent description of the structural features of the tablet composition.

In one embodiment of the invention, the pharmaceutical composition is characterized in that the composition is prepared by direct compression of a powder blend of the components of a), b) and optionally c). The direct compression of a powder blend represents a straightforward, cost-efficient, reliable and high-throughput approach towards formulation, which represents an improvement in efficiency and reliability over alternative methods of production, for example in comparison to wet granulation followed by a subsequent tabletting. In one embodiment, the direct compression of the present invention further represents an inherent description of the structural features of the tablet composition.

In one embodiment of the invention, the pharmaceutical composition is characterized in that the composition further comprises a disintegrant, a glidant and/or a lubricant.

In one embodiment of the invention, the pharmaceutical composition is characterized in that the water-soluble filler is a sugar.

In one embodiment of the invention, the pharmaceutical composition is characterized in that the water-soluble filler is mannitol.

In one embodiment of the invention, the pharmaceutical composition is characterized in that the water-soluble filler is lactose.

As described in EP0818454, known methods for manufacturing the compound bilastine can lead to the production of polymorphs 2 and/or 3 of bilastine. EP1505066 teaches subsequently the production of polymorph 1 from polymorphs 2 and/or 3 by having to conduct additional steps. The polymorph form 3 also appears to be manufactured using more straightforward techniques compared to form 1 or form 2, thereby representing an improved starting point for generating an alternative, simplified option for producing a bilastine composition. However, according to WO 2017/017301, the neat polymorphic forms 1 and 2 of bilastine are stable on storage, whilst form 3 is converted partially into the form 1 leading to a mixture of form 3 with form 1 after 1-month storage at ICH long term and accelerated conditions.

In order to simplify the production of a pharmaceutical composition comprising bilastine, the polymorph forms 2 and 3 were selected for further formulation studies, due to the absence of the requirement for further chemical processing

that would otherwise be required for the production of polymorph 1.

During the micronisation of pure polymorphic form 2, the presence of form 3 and dihydrate form A as polymorphic impurities is surprisingly observed after jet milling: two from five batches of micronized API have shown the contamination of form 2 with form 3 and dihydrate form A. As a further surprise, the XRD analysis of Bilastine form 3 API before and after micronisation shows that API remains in the polymorphic form 3 without changes during micronisation process.

Therefore, in order to simplify the production of a pharmaceutical composition comprising bilastine, the polymorph 3 was selected for further formulation studies, due to the absence of the requirement for further chemical processing that would otherwise be required for the production of polymorph 1, and as it was more stable compared to form 2 during micronisation, and as it allows greater freedom in selecting the required particle size.

As such, one object of the invention was to identify a simplified stable bilastine composition and a method of manufacture of a stable bilastine composition with suitable disintegration and dissolution properties based on polymorph form 3.

When bilastine polymorph form 3 was formulated using excipients similar to that of the reference product, the dissolution profiles were incomplete compared to the reference product, as can be determined from the dissolution studies below. Despite showing acceptable disintegration, the dissolution of formulations using polymorph form 3 and those excipients of the commercial formulation was, surprisingly, significantly retarded when compared to commercial formulations using polymorph 1.

As a further surprise, the incorporation of a water-soluble filler, in particular a sugar, more preferably mannitol or lactose, lead to significantly improved dissolution properties, achieving complete release of the API, comparable to the commercial reference product.

As a further surprise, the inventive tablet compositions prepared using the polymorphic form 3 are stable and no conversion to form 1 is observed after accelerated stability storage, despite the prior art teaching some polymorphic instability of form 3. The comparative composition containing form 2 API stored under stress conditions in the ASAP study has shown some contamination with traces Dihydrate form A, whilst the inventive composition comprising bilastine form 3 remains unchanged under the same conditions.

The incorporation of a water-soluble filler, such as the sugars listed herein, is therefore a preferred embodiment of the formulations described herein employing polymorph form 3. It was entirely unexpected that a water-soluble filler would enable improved dissolution of the API, when the presence or absence of said water-soluble filler is non-determinative for the disintegration of the tablet.

In one embodiment, the invention relates to a pharmaceutical composition as described herein in the form of a tablet, comprising a) a crystalline form 3 of bilastine, b) a water-soluble filler, preferably a sugar, such as lactose or mannitol, and c) a glidant.

In one embodiment of the invention, the pharmaceutical composition is characterized in that the glidant is silicon dioxide, preferably as colloidal anhydrous silica, magnesium aluminometasilicate and/or talc, preferably a combination of silicon dioxide and magnesium aluminometasilicate.

In a preferred embodiment, magnesium aluminometasilicate is employed, and has an unexpected stabilizing effect on the active component bilastine. In particular, the polymorphic form 3 is stabilized by magnesium aluminometasilicate in the compositions of the present invention and prevents formation of bilastine hydrates, as have been described for example in SK 7066 Y1 and WO 2017/017301.

The presence of magnesium aluminometasilicate has been shown to convey multiple benefits upon the compositions of the present inventions, and shows an unexpected synergy with bilastine. The magnesium aluminometasilicate also functions as a glidant, providing effective glidant properties improving the flowability of the blend of components prior to formulation as a tablet. Furthermore, the magnesium aluminometasilicate does not impair the improved dissolution properties of bilastine achieved by employing a water-soluble filler, as discussed in detail below. In addition, the magnesium aluminometasilicate exerts a prevention of conversion from crystalline form 3 to hydrate forms of bilastine, thereby enhancing stability of the API.

Furthermore, the improved dissolution properties of tablets comprising the water-soluble fillers as disclosed herein could be maintained over longer periods of time, thereby generating a more stable set of dissolution properties, by incorporating a glidant in the tablet, preferably selected from silicon dioxide, preferably as colloidal anhydrous silica, magnesium aluminometasilicate and/or talc. It was entirely unexpected that the incorporation of a glidant, most preferably silicon dioxide and magnesium aluminometasilicate, would enable the maintenance of the beneficial dissolution properties enabled by the water-soluble fillers over long periods of time.

As such, the combination of a water-soluble filler with a glidant as described herein, based preferably on those particular examples and specific embodiments disclosed herein, would provide a synergistic effect, i. e. the improved dissolution of the API bilastine when present as polymorph 3, combined with the prolonged stability of these dissolution properties. A skilled person would have derived neither a suggestion nor motivation from the prior art that the excipients as described herein would lead to this combination of effects.

Furthermore, these two unexpected effects lead to a set of beneficial properties of the pharmaceutical formulation that is greater than the sum of these effects when considered alone. The presence of a glidant, preferably magnesium aluminometasilicate, talc and/or silica would not necessarily provide a beneficial effect alone. The combination of a glidant with a water-soluble filler, such as a sugar, provides an unexpected enhancement of the improved dissolution.

In one embodiment of the invention, the pharmaceutical composition comprises a crystalline form 3 of bilastine, wherein the crystalline form 3 has characteristic peaks at 6.47, 12.81, 15.70, and 17.71 $\pm$ 0.2 degrees 2-theta in a powder X-ray diffraction pattern, a water-soluble filler, a water-insoluble filler, a disintegrant, a glidant, and a lubricant, wherein the water-soluble filler is D-mannitol and the glidant is colloidal anhydrous silica or a combination of colloidal anhydrous silica and magnesium aluminometasilicate.

In one embodiment of the invention, the pharmaceutical composition is characterized in that the water-insoluble filler is microcrystalline cellulose. Microcrystalline cellulose is to be considered a preferred embodiment.

In one embodiment of the invention, the pharmaceutical composition is characterized in that the disintegrant is selected from sodium starch glycolate, low-substituted hydroxypropylcellulose, pregelatinized starch, or crospovidone, preferably sodium starch glycolate or crospovidone.

In one embodiment of the invention, the pharmaceutical composition is characterized in that the lubricant is a stearate, preferably magnesium stearate.

In one embodiment of the invention, the pharmaceutical composition is characterized in that the water-soluble filler is D-mannitol, the water-insoluble filler is microcrystalline cellulose, the disintegrant is sodium starch glycolate, the glidant is colloidal anhydrous silica or a combination of colloidal anhydrous silica and magnesium aluminometasilicate, and the lubricant is magnesium stearate.

In one embodiment of the invention, the pharmaceutical composition is characterized in that the tablet is a coated or uncoated immediate-release tablet, preferably uncoated.

In one embodiment of the invention, the pharmaceutical composition comprises or consists of:

- Bilastine in an amount (wt% of the tablet) of 10-20%, preferably 12-18%, more preferably 16%;
- Mannitol in an amount of 40-70%, preferably 50-65%, more preferably 61%;
- Microcrystalline cellulose in an amount of 10-30%, preferably 12-20%, more preferably 14%;
- Sodium starch glycolate in an amount of 0.1-5%, preferably 2.5-4.5%, more preferably 4%;
- Colloidal anhydrous silica in an amount of 0.1-5%, preferably 2-4%, more preferably 2.8%; and
- Magnesium stearate in an amount of 0.1-5%, preferably 1-3%, more preferably 1.6%.

In one embodiment of the invention, the pharmaceutical composition comprises or consists of:

- Bilastine in an amount (wt% of the tablet) of 10-20%, preferably 12-18%, more preferably 16%;
- Mannitol in an amount of 40-70%, preferably 50-65%, more preferably 58%;
- Microcrystalline cellulose in an amount of 10-30%, preferably 15-25%, more preferably 20%;
- Sodium starch glycolate in an amount of 0.1-5%, preferably 0.5-2%, more preferably 1.20%;
- Colloidal anhydrous silica in an amount of 0.1-5%, preferably 0.5-2%, more preferably 0.8%;
- Magnesium aluminometasilicate in an amount of 0.1-5%, preferably 1-3%, more preferably 2%; and
- Magnesium stearate in an amount of 0.1-5%, preferably 1-3%, more preferably 1.6%.

In one embodiment of the invention, the pharmaceutical composition comprises or consists of:

- Bilastine in an amount (wt% of the tablet) of 10-20%, preferably 12-18%, more preferably 16%;
- Mannitol in an amount of 40-70%, preferably 50-65%, more preferably 61%;
- Microcrystalline cellulose in an amount of 10-30%, preferably 12-20%, more preferably 14%;

- Sodium starch glycolate in an amount of 0.1-5%, preferably 2.5-4.5%, more preferably 4%;
- Colloidal anhydrous silica in an amount of 0.1-5%, preferably 0.5-2%, more preferably 0.8%;
- Magnesium aluminometasilicate in an amount of 0.1-5%, preferably 1-3%, more preferably 2%; and
- Magnesium stearate in an amount of 0.1-5%, preferably 1-3%, more preferably 1.6%.

In a preferred embodiment the composition relates preferably to BIL/FON2, BIL/F3, BIL/F4, and BIL/F6 or alternative formulations based closely on these embodiments, as demonstrated below, in particular compositions with quantities of components falling within the ranges indicated by the "% range" parameters disclosed below.

The below embodiments may also be considered to encompass further embodiments of the invention in which the indicated amounts of the components are employed, but alternative water-soluble fillers, water insoluble fillers, disintegrants, glidants and/or lubricants, other than the specific components disclosed below, are used.

**Embodiments based on BIL/FON2:**

| **S.No.** | **Ingredient** | **mg/tab** | | | |
|---|---|---|---|---|---|
| | **Stage-A (Blending and Dry granulating)** | **Function** | **mg/tab** | **wt%** | **Preferred % range** |
| 1 | Bilastine (In house) | API | 20,05 | 16,04% | 10-20 |
| 2 | Mannitol Ph. Eur. (Pearlitol® 200 SD) | Water-soluble filler | 76,50 | 61,20% | 40-70 |
| 3 | Cellulose, Microcrystalline Ph. Eur. (Avicel® PH 200) | Water-insoluble filler | 17,95 | 14,36% | 10-30 |
| 4 | Sodium Starch Glycolate Ph. Eur. (Explotab®) | Disintegrant | 5,00 | 4,00% | 0.1-5 |
| 5 | Silica, colloidal anhydrous Ph. Eur. (Aerosil® 200 Pharma) | Glidant | 2,50 | 2,00% | 0.1-5 |
| 6 | Magnesium Stearate Ph. Eur. (Valtris) | Lubricant | 1,00 | 0,80% | 0.1-5 |
| | **Stage-B (Lubrication)** | | | | |
| 1 | Silica, colloidal anhydrous Ph. Eur. (Aerosil® 200 Pharma) | Glidant | 1,00 | 0,80% | 0.1-5 |
| 2 | Magnesium Stearate Ph. Eur. (Valtris) | Lubricant | 1,00 | 0,80% | 0.1-5 |
| | **Tablet Weight (mg):** | | **125.00** | 100.00% | 100.00% |

**Embodiments based on BIL/F3 and BIL/F4:**

| **S.No.** | **Ingredient** | **mg/tab** | | | |
|---|---|---|---|---|---|
| | **Stage-A (Blending and Dry granulating)** | **Function** | **mg/tab** | **wt%** | **Preferred % range** |
| 1 | Bilastine (In house) | API | 20,09 | 16,07% | 10-20 |
| 2 | Mannitol Ph. Eur. (Pearlitol® 200 SD) | Water-soluble filler | 73,00 | 58,40% | 40-70 |
| 3 | Cellulose, Microcrystalline Ph. Eur. (Avicel® PH 200) | Water-insoluble filler | 24,91 | 19,93% | 10-30 |
| 4 | Sodium Starch Glycolate Ph. Eur. (Explotab®) | Disintegrant | 1,50 | 1,20% | 0.1-5 |
| 5 | Silica, colloidal anhydrous Ph. Eur. (Aerosil® 200 Pharma) | Glidant | 0,50 | 0,40% | 0.1-5 |
| 6 | Magnesium Aluminometasilicate Ph. Eur. (Neusilin® US2) | Glidant | 2,50 | 2,00% | 0.1-5 |

(continued)

| S.No. | Ingredient | mg/tab | | | |
|---|---|---|---|---|---|
| | **Stage-A (Blending and Dry granulating)** | **Function** | **mg/tab** | **wt%** | **Preferred % range** |
| 7 | Magnesium Stearate Ph. Eur. (Valtris) | Lubricant | 1,00 | 0,80% | 0.1-5 |
| | **Stage-B (Lubrication)** | | | | |
| 1 | Silica, colloidal anhydrous Ph. Eur. (Aerosil® 200 Pharma) | Glidant | 0,50 | 0,40% | 0.1-5 |
| 2 | Magnesium Stearate Ph. Eur. (Valtris) | Lubricant | 1,00 | 0,80% | 0.1-5 |
| | **Tablet Weight (mg):** | | **125.00** | 100.00% | 100.00% |

**Embodiments based on BIL/F6:**

| S.No. | Ingredient | mg/tab | | | |
|---|---|---|---|---|---|
| | **Stage-A (Blending and Dry granulating)** | **Function** | **mg/tab** | **wt%** | **Preferred % range** |
| 1 | Bilastine (In house) | API | 20,05 | 16,04% | 10-20 |
| 2 | Mannitol Ph. Eur. (Pearlitol® 200 SD) | Water-soluble filler | 76,50 | 61,20% | 40-70 |
| 3 | Cellulose, Microcrystalline Ph. Eur. (Avicel® PH 200) | Water-insoluble filler | 17,95 | 14,36% | 10-30 |
| 4 | Sodium Starch Glycolate Ph. Eur. (Explotab®) | Disintegrant | 5,00 | 4,00% | 0.1-5 |
| 5 | Magnesium Aluminometasilicate Ph. Eur. (Neusilin® US2) | Glidant | 2,50 | 2,00% | 0.1-5 |
| 6 | Magnesium Stearate Ph. Eur. (Valtris) | Lubricant | 1,00 | 0,80% | 0.1-5 |
| | **Stage-B (Lubrication)** | | | | |
| 1 | Silica, colloidal anhydrous Ph. Eur. (Aerosil® 200 Pharma) | Glidant | 1,00 | 0,80% | 0.1-5 |
| 2 | Magnesium Stearate Ph. Eur. (Valtris) | Lubricant | 1,00 | 0,80% | 0.1-5 |
| | **Tablet Weight (mg):** | | **125.00** | 100.00% | 100.00% |

In the potential embodiments based on BIL/FON2, BIL/F3, BIL/F4, and BIL/F6, the total amount of glidant and/or lubricant, whether added in the stage A and/or stage B, is preferably 0.1-5% based on the total weight of all components of the tablet.

The invention further relates to a pharmaceutical composition as described herein for use as a medicament in the treatment of allergic rhino-conjunctivitis and/or urticaria, comprising administering to a patient in need thereof a pharmaceutical composition in accordance with the composition described herein, preferably a composition comprising a therapeutically relevant or efficacious amount of bilastine.

The invention further relates to a method of preparing a pharmaceutical composition in the form of a tablet, comprising dry granulation of a blend and compression of the granules to a tablet, wherein the blend comprises a) a crystalline form 3 of bilastine, wherein the crystalline form 3 has characteristic peaks at 6.47, 12.81, 15.70, and 17.71 $\pm$ 0.2 degrees 2-theta in a powder X-ray diffraction pattern, b) a water-soluble filler and optionally c) a water-insoluble filler.

In a preferred embodiment, the method comprises blending of the components a), b) and optionally c), roller compaction and granulation of the blend, milling the granules, lubricating the granules and subsequent compression of the granules to a tablet.

A dry granulation method is surprisingly advantageous with respect to minimizing the impact of API variability on processing.

The invention further relates to a method of preparing a pharmaceutical composition in the form of a tablet,

comprising direct compression of a powder blend, wherein said powder blend comprises a) a crystalline form 3 of bilastine, wherein the crystalline form 3 has characteristic peaks at 6.47, 12.81, 15.70, and 17.71 ± 0.2 degrees 2-theta in a powder X-ray diffraction pattern, b) a water-soluble filler and optionally c) a water-insoluble filler.

As shown in more detail below, in the form of examples, the method of the present invention enables a straightforward, cost-efficient, reliable and high-throughput approach towards formulation of bilastine, which represents an improvement in efficiency and reliability over alternative methods of production. The method is capable of producing stable bilastine formulations, with excellent content uniformity within the specification limits according to European Pharmacopeia 2.9.40 independent of changes in the blending parameters (pre-lubrication time, lubrication time, and blender speed).

In some embodiments, wet granulation or other methods involving processing components with moisture are not employed. Dry methods of production, such as dry granulation or direct compression are preferred. As shown below, experiments have been performed using the wet granulation methods and compositions described in CN106692090A. These experiments reveal that wet granulation of either the polymorphic form 2 or 3 in the compositions as described therein leads to conversion to "Form Alpha" as described in WO 2017/017301, otherwise known as "Dihydrate Form A". Furthermore, wet granulation appears to have a detrimental effect on the dissolution properties of the API.

In one embodiment of the invention, the method for preparing the pharmaceutical composition is characterized in that the method comprises:

- Dispensing the blend components, preferably comprising a) a crystalline form 3 of bilastine, wherein the crystalline form 3 has characteristic peaks at 6.47, 12.81, 15.70, and 17.71 ± 0.2 degrees 2-theta in a powder X-ray diffraction pattern, b) a water-soluble filler and optionally c) a water-insoluble filler;
- Sifting bilastine and the filler(s) as a blend through a suitable mesh;
- Optionally sifting the disintegrant, glidant(s) and lubricant separately through a suitable mesh /this step may also be combined with the step above, enabling a single sifting step);
- Loading the materials above (preferably with the exception of the lubricant or with only a portion of the lubricant) in a blender and mixing for sufficient time to mix the components (pre-lubrication blending);
- Dry granulation of the blend, preferably using roller compaction;
- Optionally milling the granules in order to achieve the desired granule size;
- Optionally adding the lubricant (if not already incorporated, or adding the remaining portion of the lubricant) or a combination of lubricant with glidant(s), and continue the blending for sufficient time to mix the granules with the lubricant or the combination of lubricant with glidant(s) (lubrication blending);
- Compressing the granules into tablets using the (lubricated) granules of the previous step.

In one embodiment of the invention, the method for preparing the pharmaceutical composition is characterized in that the method comprises:

- Dispensing the blend components, preferably comprising a) a crystalline form 3 of bilastine, wherein the crystalline form 3 has characteristic peaks at 6.47, 12.81, 15.70, and 17.71 ± 0.2 degrees 2-theta in a powder X-ray diffraction pattern, b) a water-soluble filler and optionally c) a water-insoluble filler;
- Sifting bilastine and the filler(s) as a blend through a suitable mesh;
- Optionally sifting the disintegrant, glidant(s) and lubricant separately through a suitable mesh;
- Loading the materials above, preferably with the exception of the lubricant, in a blender and mixing for sufficient time to mix the components (pre-lubrication blending);
- Optionally adding the lubricant (if not already incorporated) and continue the blending for sufficient time to mix the components (lubrication blending);
- Compressing the tablets using the blend of the previous step.

In one embodiment of the invention, the method for preparing the pharmaceutical composition is characterized in that the pre-lubrication blending is carried out for 1-60 minutes, preferably 5-30 minutes, more preferably 8-25 minutes, in particular 10, 15 or 20 minutes.

In one embodiment of the invention, the method for preparing the pharmaceutical composition is characterized in that the lubrication blending is carried out for 0-30 minutes, preferably 1-10 minutes, more preferably 2-8 minutes, in particular 3, 5 or 7 minutes.

In one embodiment of the invention, the method for preparing the pharmaceutical composition is characterized in that the blending is conducted in a blender with 5-100 rpm, preferably 5-40 rpm, in particular 10, 20 or 30 rpm blending speed.

## DETAILED DESCRIPTION OF THE INVENTION

"Bilastine" or (name 2-[4-(2-(4-(1-(2-ethoxyethyl)-1H-benzimidazol-2-yl)piperidin-1-yl)ethyl)phenyl]-2-methylpropionic acid), recorded under CAS number 202189-78-4 is known as a second generation antihistamine drug for the treatment of allergic rhinoconjunctivitis and urticaria (hives). It exerts its effect as a selective histamine H1 receptor antagonist, and has an effectiveness similar to cetirizine, fexofenadine and desloratadine. Bilastine is approved in the European Union for the symptomatic treatment of allergic rhinoconjunctivitis and urticaria.

The term "bilastine" as used herein according to the present invention includes bilastine in the form of free base, a pharmaceutically acceptable salt thereof, amorphous bilastine, crystalline bilastine, preferably selected from the polymorph forms described herein, any isomer, derivative, hydrate, solvate or prodrug or a combination thereof.

Bilastine or polymorphs thereof may also be prepared as a pharmaceutical salt. Examples of pharmaceutical acceptable salts of bilastine which can be contained as an active ingredient in a solid oral dosage form include acid addition salt formed with mineral acids such as hydrochloric acid, hydrobromic acid, hydroiodic acid, sulfuric acid, nitric acid, phosphoric acid and the like; acid addition salts formed with organic acids such as acetic acid, propionic acid, butyric acid, oxalic acid, citric acid, succinic acid, tartaric acid, fumaric acid, malic acid, lactic acid, adipic acid, benzoic acid, salicylic acid, methanesulfonic acid, benzenesulfonic acid, p-toluenesulfonic acid, glutamic acid, aspartic acid and the like. Examples of solvate include solvates with water, ethyl alcohol or the like.

The polymorph forms 1, 2 and 3 of bilastine are known from the prior art, for example in the descriptions of EP1505066, WO 2014/026657 and WO 2017/017301.

All references to the "polymorph I form", "polymorph 1 form", "polymorph 1", "polymorph I", "form 1" or "form I", "crystalline form 1" or "crystalline form I" or the like may be used interchangeably.

Form 1, described in example 12 of WO 2014026657 relates to a crystalline form with the following characteristics:

XRPD ([° 2Th.] (rel. int %)): 3.64 (4.4), 10.57 (23.3), 11.27 (78.1), 12.47 (38.8), 14.08 (26.9), 15.07 (38.4), 15.50 (16.5), 16.27 (43.6), 17.16 (100.0), 18.89 (71.8), 19.73 (74.0), 21.13 (33.9), 22.17 (18.1), 22.71 (26.9), 23.34 (10.3), 24.88 (18.6), 25.82 (9.2), 26.58 (1 1.5), 28.43 (9.7), 29.16 (8.8), 30.92 (4.6), 34.38 (9.5), 37.01 (5.4).

The figure 1 of WO2017/017301 shows the XRD pattern of form 1 with the same peak positions as the pattern of form 1 disclosed in the figure 7 of WO 2014/026657.

The inventors have also analysed bilastine form 1 and find the characteristic diffraction peaks to be present at 10.57, 11.27, 12.44, 14.08, 15.07, 15.50, 16.27, and 17.16 $\pm$ 0.2° 2Theta.

For the monitoring of bilastine form 1 presence in the tablets, the most suitable peaks are the characteristic and non-interfering reflexes at 12.47 and/or 14.08 $\pm$ 0.2° 2Theta.

Dihydrate form A of bilastine is known from the prior art, for example in the description of SK 7066 Y1. According to SK 7066 Y1, dihydrate form A has characteristic peaks at 8.1, 11.5, 13.8, 17.6, 20.0, 21.1, and 23.2 $\pm$ 0.2 degrees 2-theta in a powder X-ray diffraction pattern. According to table 1 of SK 7066 Y1, the following relative intensity is measured at these positions: XRPD ([° 2Th.] (rel. int. %)): 8.26 (10.5), 8.06 (100.0), 10.86 (20.8), 11.50 (45.7), 12.15 (20.2), 13.84 (50.5), 17.59 (77.4), 18.52 (47.0), 18.76 (41.0), 20.04 (33.9), 21.08 (32.1), 23.22 (19.6), 26.17 (17.5).

However, by analysis of the dihydrate form A by the present inventors, it appears a mistake is evident in the list provided in SK 7066 Y1. There is no peak in XRD pattern visible at 8.1. Based on d-spacing of 10.155, the calculated position should be 8.70° 2Th. This value is in line with the measurements of the present inventors, where no peak is present at 8.1, but a strong peak is visible at ca. 8.7° 2Th.

WO 2017/017301 (Urquima) discloses the preparation of hydrate form Alpha of bilastine and its XRD, IR, and DSC data. The XRD pattern of hydrate form Alpha in WO 2017/017301 is essentially the same as the XRD pattern of dihydrate form A, disclosed in SK 7066 Y1 (Zentiva), leading to the conclusion that "hydrate Alpha" is the same form as "dihydrate form A", only with a different name.

According to the present invention, dihydrate form A has characteristic peaks at 8.7, 11.5, 13.8, 17.6, 20.0, 21.1, and 23.2 $\pm$ 0.2 degrees 2-theta in a powder X-ray diffraction pattern. Furthermore, Dihydrate form A has additional characteristic peaks at 8.3, 10.9, 12.2, 18.5, 18.8, and 26.2 $\pm$ 0.2 degrees 2-theta in a powder X-ray diffraction pattern.

The following relative intensity is measured at these positions:
XRPD ([° 2Th.] (rel. int. %)): 8.26 (10.5), 8.70 (100.0), 10.86 (20.8), 11.50 (45.7), 12.15 (20.2), 13.84 (50.5), 17.59 (77.4), 18.52 (47.0), 18.76 (41.0), 20.04 (33.9), 21.08 (32.1), 23.22 (19.6), 26.17 (17.5).

The inventors have prepared bilastine dihydrate form A by water slurry of Bilastine Form 2 for 1 day at room temperature or 40°C. Alternatively, dihydrate form A is prepared by water slurry of Bilastine Form 3 for 1 day at 40°C.

The inventors analysed bilastine dihydrate form A and find the most characteristic peaks to be present at 8.7, 12.2, 13.8, and 17.6 ± 0.2 degrees 2-theta in a powder X-ray diffraction pattern.

In tablets containing bilastine crystalline form 2 and traces of bilastine dihydrate form A, only the non-interfering peak of dihydrate form A at 8.7 ± 0.2 degrees 2-theta is detectable in the powder X-ray diffraction pattern among the reflexes of bilastine crystalline form 2 and excipients.

In tablets containing bilastine crystalline form 2 and a minor amount of bilastine dihydrate form A, only non-interfering peaks of dihydrate form A at 8.7 and 12.2 ± 0.2 degrees 2-theta are detectable in the powder X-ray diffraction pattern among the reflexes of bilastine crystalline form 2 and excipients.

All references to the "polymorph II form", "polymorph 2 form", "polymorph 2", "polymorph II", "form 2" or "form II", "crystalline form 2" or "crystalline form II" or the like may be used interchangeably.

Form 2, described in example 12 of WO 2014026657 relates to a crystalline form with the following characteristics:
XRPD ([° 2Th.] (rel. int %)): 6.53 (100.0), 9.43 (30.8), 11.04 (22.8), 13.39 (6.2), 15.24 (32.2), 15.86 (86.1), 18.07 (29.9), 18.39 (36.2), 18.94 (8.3), 20.19 (16.0), 20.66 (19.0), 21.70 (17.1), 22.17 (15.6), 23.70 (5.7), 26.59 (4.9), 28.03 (3.6), 28.33 (3.6), 29.70 (4.3).

The figure 4 of WO2017/017301 shows the XRD pattern of form 2 with essentially the same peak positions as the pattern of form 2 disclosed in the figure 9 of WO 2014/026657.

The inventors have also analysed bilastine form 2 and find the characteristic diffraction peaks to be present at 6.53°, 15.24°, 15.86°, and 18.07° ± 02 degrees 2-theta.

In some embodiments, two additional reflexes (characteristic diffraction peaks) at 9.43° and 11.04° ± 0.2 degrees 2-theta, which are also characteristic and well visible in the XRPD analyses conducted despite signals from placebo, may be employed for a definition of the crystalline form 2.

Preferably, characteristic reflexes for Form 2 are 6.53°, 9.43°, 11.04, 15.24°, 15.86°, and 18.07° ± 0.2° 2Theta.

The powder X-ray diffraction pattern of tablets containing only bilastine form 2 as API shows characteristic peaks at 6.53°, 15.24°, 15.86°, and 18.07° ± 0.2° 2Theta, whilst no characteristic peaks are observed for form 1, for example at 12.47 and/or 14.08 ± 0.2° 2Theta, and for dihydrate form A, for example at 8.7 ± 0.2° 2Theta (and optionally at 12.2 ± 0.2° 2Theta).

In embodiments of the present invention, the polymorph 3 form is preferred. All references to the "polymorph III form", "polymorph 3 form", "polymorph 3", "polymorph III", "form 3" or "form III", "crystalline form 3" or "crystalline form III" or the like may be used interchangeably.

Figure 7 of WO 2017/017301 discloses the XRD pattern of polymorphic form 3 of bilastine. According to WO 2017/017301, the neat polymorphic forms 1 and 2 of bilastine and hydrates Alpha and Eta are stable on storage, whilst form 3 is converted partially into the form 1 leading to a mixture of form 3 with form 1 after 1 month storage at 25°C/60%RH (ICH long term conditions) and at 40°C/75%RH (ICH accelerated conditions), and the phase transformation is increasing further after 2 months under accelerated conditions to afford a mixture of form 1 with form 3.

IPCOM000247653D disclosed the XRD data of so called "form K3" of bilastine. The XRD pattern of form K3 in the figure 11 of IPCOM000247653D is essentially the same as the XRD pattern of form 3, disclosed in figure 7 of WO 2017/017301, leading to the conclusion that "form K3" is the same form as "form 3", only with a different name. According to IPCOM000247653D, this form of bilastine is characterized by a short list of 3 characteristic peaks at 15.7, 17.7, and 20.2 ± 0.2° 2-theta or by a preferred longer list of 10 peaks at 6.5, 9.3, 10.9, 12.8, 15.7, 17.7, 18.4, 20.2, 22.0, and 27.4 ± 0.2° 2Theta.

The inventors have also analysed bilastine form 3 and find the characteristic diffraction peaks to be present at 6.47, 9.29, 10.92, 12.81, 15.70, 17.71, 18.38, 20.17, 21.97, and 27.36 ± 0.2° 2Theta in a powder X-ray diffraction pattern.

Preferably, characteristic reflexes for form 3 are 6.47, 12.81, 15.70, and 17.71 ± 0.2° 2Theta.

The powder X-ray diffraction pattern of tablets containing only bilastine form 3 as API is showing characteristic peaks at 6.47, 12.81, 15.70, and 17.71 ± 0.2° 2Theta, whilst no characteristic peaks are observed for form 1, for example at 12.47 and/or 14.08 ± 0.2° 2Theta, and for dihydrate form A, for example at 8.7 ± 0.2° 2Theta (and optionally at 12.2 ± 0.2° 2Theta).

The term "essentially the same" with reference to PXRD means that variabilities in peak positions and relative intensities of the peaks are to be taken into account. For example, a typical precision of the 2-Theta values is in the range of ± 0.2° 2-Theta. Thus, for example a diffraction peak that usually appears at 15.7° 2-Theta for example can appear between 15.5° and 15.9 ° 2-Theta on most X-ray diffractometers under standard conditions. With respect to the relative intensities and the characteristic peaks of the powder X-ray diffraction patterns mentioned above, the provided

values of relative intensity are not intended as limiting for the identification of the characteristic peaks mentioned. As is known to a skilled person, the relative peak intensities will show inter-apparatus variability, batch-to-batch variability, as well as variability due to degree of crystallinity, preferred orientation, sample preparation, and as such are provided as an indication and as qualitative measure only, but not a limiting definition, of the intensities of the peaks in the powder X-ray diffraction patterns. The term "characterizing peak" in the context of defining the present invention is therefore not limited to the respective relative intensities provided above, and any one or more of the respective peaks may be determined as a characterizing peak for any given form of bilastine. Preferably at least 1, 2, 3 or 4 peaks are used to characterize a bilastine polymorphic form, in other embodiments, at least 5, 6, 7, 8 9 or 10 peaks may be employed.

The term "active ingredient" or "API" herein refers to a pharmaceutically active molecule (e.g. bilastine) as well as its pharmaceutically acceptable and therapeutically active salts, esters, amides, prodrugs, metabolites, enantiomers, polymorphs, analogs, etc. that induce a desired pharmacological or physiological effect. Terms like "active", "active agent", "active substance" may be used synonymously for "active ingredient".

The term "effective amount" or "therapeutically effective amount" used interchangeably, is defined to mean the amount or quantity of the active drug (e.g. bilastine), which is sufficient to elicit an appreciable biological response when administered to the patient. It will be appreciated that the precise therapeutic dose will depend on the age and condition of the patient, nature of the condition to be treated and will be at the ultimate discretion of the attendant physician.

The term "excipient" means a pharmacologically inactive component such as a diluent, disintegrant, carrier, and the like, of a pharmaceutical product. The excipients that are useful in preparing a pharmaceutical composition are generally safe, non-toxic and are acceptable for veterinary as well as human pharmaceutical use. Reference to an excipient includes both one excipient and more than one excipient.

The excipients are described herein in some embodiments according to "wt%", or "percentage by weight". The %wt values recite herein preferably relate to the percentage of material by weight present in the tablet, or in the powder blend prior to compression.

According to the invention, a water-soluble filler may be used, i.e. as a bulking agent. Various useful water-soluble fillers include sugars, such as mannitol, lactose, sorbitol, xylitol, and the like, and mixtures thereof; more preferably selected from mannitol and lactose.

According to the invention, a water-insoluble filler may be used. Various useful water-insoluble fillers include starch, powdered cellulose, microcrystalline cellulose (MCC), calcium phosphate and the like, or combinations thereof.

According to the present invention, one or more lubricants can be used. Useful lubricants include stearates, such as magnesium stearate, or sodium stearyl fumarate.

According to the present invention, one or more glidants can be used. Useful glidants include silica, in various forms, such as colloidal silicon dioxide, magnesium aluminometasilicate (also known as Neusilin®), magnesium silicate, magnesium trisilicate, talc, and other forms of silicon dioxide, such as aggregated silicates and hydrated silica.

Magnesium aluminometasilicate (also known as Silodrate or Simaldrate) typically occurs as a white powder, grain, or granule. It is commonly known according to the following formula $Al_2O_2.2Mg.3O_3Si$ with a MW of 362.821 g/mol. Magnesium aluminometasilicate may also be identified with the following formula $Al_2H_2Mg_2O_{12}Si_3$ with a MW of 380.832 g/mol. Magnesium aluminometasilicate may also be identified with the following formula $Al_2O_3.MgO.1.7SiO_2.xH_2O$ (CAS number 12511-31-8). In a preferred embodiment, the magnesium aluminometasilicate is employed as Neusilin®, which is a fine granule of magnesium aluminometasilicate.

According to the present invention, a disintegrant is typically an agent used in the preparation of solid pharmaceutical formulations which causes them to disintegrate and release their medicinal substances on contact with moisture. Disintegrants include hydroxypropyl cellulose (L-HPC), pregelatinized starch (PGS), crospovidone, croscarmellose sodium, sodium starch glycolate and the like.

The composition may also comprise other excipients, such as surfactants and/or binders, as may be desired.

A "tablet" as used herein is considered a solid unit dosage form of a medicament comprising one or more excipients.

The most commonly used pharmaceutical solid dosage forms today include granules, pellets, tablets and capsules. Tablets are solid pharmaceutical dosage forms containing drug substances with one or more excipients prepared by either compression or molding methods. The basic art of tableting by three well known methods includes direct compression, wet granulation and dry granulation. According to the present invention dry granulation and direct compression are preferred. Dry granulation may typically be employed if the materials have sufficient inherent binding or cohesive properties to form granules. Dry granulation refers typically to the process of granulating without the use of liquids. Two dry granulation methods are primarily used in the pharmaceutical industry, namely slugging and roller compaction. In a roller compactor material particles are consolidated and densified by passing the material between two high pressure rollers. The densified material from a roller compactor is then reduced to a uniform granule size by milling. Roll compaction/dry granulation (RCDG) is a method of choice for processing of physically or chemically moisture sensitive drugs, as no liquid binder is required in the granulation, and is a preferred method of the present invention.

In the dry granulation method of tablet production, dry ingredients are thoroughly mixed to a blend, the blend

is granulated, for example by roller compaction and granulation and optionally milling, and the granules are then compressed into tablets. This eliminates the drying steps associated with the wet granulation method. It also reduces the higher costs involved in wet granulation including increased equipment, labor, time, process validation and energy expenditure. As a result, dry granulation is both efficient and economical, well suited to the production of high quality tablets, which exhibit hardness, low friability and excellent dissolution rates. As an added benefit, dry granulation can avoid the undesired polymorphic conversion of anhydrous solid form of API to a hydrate form during the tablet manufacturing, and it can also improve the physical and chemical stability of tablets as compared to wet granulation.

In the direct compression method of tablet production, dry ingredients are thoroughly mixed to a powder blend and then compressed into tablets. This eliminates the drying steps associated with the wet granulation method. It also reduces the higher costs involved in wet granulation including increased equipment, labor, time, process validation and energy expenditure. As a result, direct compression is both efficient and economical, well suited to the production of high quality tablets, which exhibit hardness, low friability and excellent dissolution rates. As an added benefit, direct compression can avoid the undesired polymorphic conversion of anhydrous solid form of API to a hydrate form during the tablet manufacturing, and it can also improve the physical and chemical stability of tablets as compared to wet granulation.

According to the invention, a powder or powder blend is compressed, which is a substantially dry solid, composed of preferably a large number of fine particles that may flow when shaken or tilted. The powder of the present invention is preferably distinguishable from granules, which are typically prepared by wet or dry granulation, and are typically larger than the particles of the powder of the invention. According to one embodiment of the invention, the powder blend is directly compressed.

Methods for compression of tablets are known to a skilled person and may be elected appropriately without undue effort. For example, after mixing of ingredients (in case of direct compression), the powder blend may be directly compressed to obtain a tablet. Methods for compression of granules into tablets are also known to a skilled person, wherein granules are subsequently compressed into tablets. The compression is preferably carried out by either by single punch machine (stamping press) or by multi station machine (rotary press). The tablet press is a high-speed mechanical device. It ’squeezes’ the ingredients into the required tablet shape with extreme precision. It can make the tablet in many shapes, although they are usually round or oval. Also, it can press the name of the manufacturer or the product into the top of the tablet. Allergic rhinoconjunctivitis, or Rhinitis, or Allergic rhinitis, or hay fever, is typically caused by an inflammation in the nose which occurs when the immune system overreacts to allergens in the air. Rhinitis is characterized by nasal congestion, runny nose, post-nasal drip, sneezing, red eyes and/or itching of the nose or eyes. Allergic rhinitis is typically associated with the presence of antibodies (IgE) which recognize certain allergens. When these antibodies are exposed to the allergen, they bind the allergen and this leads to an inflammatory reaction. This reaction is characterized by the liberation of several inflammatory mediators, which in turn leads to the signs and symptoms typical of allergic rhinitis.

Urticaria, or hives, is a vascular reaction of the skin marked by the transient appearance of smooth plaques (wheals). Individual lesions typically resolve without scarring in several hours.

Most cases of urticaria are self-limited and of short duration; the eruption rarely lasts more than several days, it but may be recurrent over weeks. Chronic urticaria is also treatable using the API as described herein. A medical practitioner is capable of electing a suitable dose of the tablet described herein for treatment of allergic rhinoconjunctivitis or urticaria, depending on the age and size of the patient, and severity of disease, or other relevant factors.

**FIGURES**

The invention is demonstrated by way of the figures disclosed herein. The figures provide support for a detailed description of potentially preferred embodiments of the invention.

Fig. 1 shows the powder XRD of Form 1 Bilastine API.
Fig. 2 shows the powder XRD of Form 2 Bilastine API (unmicronized API, "as is" from the synthesis)
Fig. 3 shows the powder XRD of Form 3 Bilastine API (unmicronized API, "as is" from the synthesis)
Fig. 4 shows the powder XRD of Form 3 Bilastine API micronized by jet-mill to $D_{90}$ (Malvern, by volume) of 8.14 micron
Fig. 5 shows the powder XRD of Dihydrate form A of Bilastine API.
Fig. 6 shows the powder XRD of tablets BIL/CX (Initial).
Fig. 7 shows the powder XRD of tablets BIL/CX (40°C/75%RH, 1 month in Alu-Alu blister).
Fig. 8 shows the powder XRD of tablets BIL/CX (50°C/75%RH 14 Days open exposure).
Fig. 9 shows the powder XRD of tablets BIL/F3 (40°C/75%RH, 1 month in Alu-Alu blister).
Fig. 10 shows the powder XRD of tablets BIL/F6 (Initial).
Fig. 11 shows the powder XRD of tablets BIL/F6 (40°C/75%RH, 2 months in Alu-Alu blister).
Fig. 12 shows the powder XRD of tablets BIL/F6 (50°C/75%RH 14 Days open exposure).

## EXAMPLES

The invention is demonstrated by way of the examples disclosed herein. The examples provide technical support for a detailed description of potentially preferred embodiments of the invention.

**General procedure for the preparation of examples below employing a direct compression process:**

1.0 Dispense the materials according to the indicated amounts.
2.0 Sift Bilastine and Filler(s) through suitable mesh.
3.0 Sift Disintegrant, Glidant(s) and Lubricant separately through suitable mesh.
4.0 Load the materials from steps 2.0 and 3.0 except Lubricant in blender and mix for sufficient time.
5.0 Add Lubricant from Step 2.0 to 4.0 and continue the blending for sufficient time.
6.0 Compress the tablets using blend from step 5.0.

**General procedure for the preparation of examples below employing a dry granulation process:**

1.0 Dispense the materials according to the indicated amounts.

2.0 Sift Bilastine and Filler(s) through suitable mesh.

3.0 Sift Disintegrant, Glidant(s) and Lubricant separately through suitable mesh.

4.0 Load the Stage-A materials from the above steps 2.0 and 3.0 in blender and mix for sufficient time.

5.0 The blend from step 4.0 is roll compacted to prepare the granules.

6.0 The compacts are milled using Quadro® Comil® to get suitable sized granules.

7.0 Load the granules from Step 6.0 into the blender. Add Stage-B excipients (e.g. second portion of sifted Magnesium Stearate and optionally Colloidal Anhydrous Silica) into the blender and continue the blending for sufficient time.

8.0 Compress the tablets using lubricated blend from step 7.0.

**<u>Part A - Comparative examples</u>**

**Comparative example BIL/CE1 (direct compression process):**

| **S.No.** | **Ingredient** | **mg/tab** | |
|---|---|---|---|
| | **Stage-A (Sifting & Blending)** | **Function** | **mg/tab** |
| 1 | Bilastine, Form 3 *(In house)* | API | 20.050 |
| 2 | Cellulose, Microcrystalline *Ph. Eur.* (Avicel® PH 200) | Water-insoluble filler | 102.950 |
| 3 | Sodium Starch Glycolate *Ph. Eur.* (Explotab®) | Disintegrant | 1.000 |
| 4 | Silica, colloidal anhydrous *Ph. Eur.(Aerosil*® 200) | Glidant | 0.960 |
| | **Stage-B (Lubrication)** | | |
| 1 | Magnesium Stearate *Ph. Eur.*(Ferro) | Lubricant | 1.940 |
| | **Tablet Weight (mg):** | | **126.900** |

**Comparative example BIL/CE2 (dry granulation process):**

| S.No. | Ingredient | mg/tab | |
|---|---|---|---|
| | **Stage-A (Blending&Compaction)** | **Function** | **mg/tab** |
| 1 | Bilastine, Form 3 *(In house)* | API | 20.050 |
| 2 | Cellulose, Microcrystalline *Ph. Eur.* (Avicel® PH 200) | Water-insoluble filler | 101.990 |
| 3 | Sodium Starch Glycolate *Ph. Eur.* (Explotab®) | Disintegrant | 1.000 |
| 4 | Magnesium Stearate *Ph. Eur.* (Ferro) | Lubricant | 0.500 |
| | **Stage-B (Lubrication)** | | |
| 1 | Silica, colloidal anhydrous *Ph. Eur.* (Aerosil® 200) | Glidant | 0.960 |
| 2 | Magnesium Stearate *Ph. Eur.* (Ferro) | Lubricant | 1.940 |
| | **Tablet Weight (mg):** | | **126.440** |

**Comparative example BIL/CE3 (direct compression process):**

*This examole is corresoonding essentially to Examole 10D of* WO 2017/017301, *which emoloys the polymorohic form 2 of Bilastine, whilst form 3 is used below:*

| S.No. | Ingredient | mg/tab |
|---|---|---|
| | **Stage - A (Sifting & Blending)** | **mg/tab** |
| 1 | Bilastine, Form 3 (*In house*) | 20.050 |
| 2 | Cellulose, Microcrystalline *Ph.Eur.* (Avicel® PH 200) | 98.070 |
| 3 | Sodium Starch Glycolate *Ph.Eur.* (Explotab®) | 5.000 |
| 4 | Silica, colloidal anhydrous *Ph.Eur.* (Aerosil® 200 Pharma) | 0.630 |
| | **Stage - B (Lubrication)** | |
| 1 | Magnesium Stearate *Ph. Eur.* | 1.250 |
| | **Tablet Weight (mg):** | **125.000** |

**Comparative example BIL/CE4 ((direct compression process, using Form 2 API):**

*This comoarative example is the re-work to Example 10D of* WO 2017/017301, *which emoloys the polymorphic form 2 of Bilastine.*

| S.No. | Ingredient | mg/tab |
|---|---|---|
| | **Stage - A (Sifting & Blending)** | **mg/tab** |
| 1 | Bilastine, Form 2 (*In house*) | 20.090 |
| 2 | Cellulose, Microcrystalline *Ph.Eur.* (Comprecel® M102 D+) | 98.030 |
| 3 | Sodium Starch Glycolate *Ph.Eur.* (Explotab®) | 5.000 |
| 4 | Silica, colloidal anhydrous *Ph.Eur.* (Aerosil® 200 Pharma) | 0.630 |
| | **Stage - B (Lubrication)** | |
| 1 | Magnesium Stearate *Ph. Eur.* | 1.250 |
| | **Tablet Weight (mg):** | **125.000** |

**Additional example:**

**Example BIL/CX (direct compression process, using Form 2 API)**

| S.No. | Ingredient | mg/tab | |
|---|---|---|---|
| | **Stage-A (Sifting & Blending)** | **Function** | **mg/tab** |
| 1 | Bilastine, Form 2 (*In house*) | API | 20.149 |
| 2 | Mannitol *Ph. Eur.* (Pearlitol® 200 SD) | Water-soluble filler | 73.000 |
| 3 | Cellulose, Microcrystalline *Ph. Eur.* (Avicel® PH 200) | Water-insoluble filler | 28.601 |
| 4 | Crospovidone *Ph. Eur.* (Polyplasdone® XL-10) | Disintegrant | 0.500 |
| 5 | Silica, colloidal anhydrous *Ph. Eur.* (Aerosil® 200) | Glidant | 1.000 |
| | **Stage-B (Lubrication)** | | |
| 1 | Magnesium Stearate *Ph. Eur.* (Ferro) | Lubricant | 1.750 |
| | **Tablet Weight (mg):** | | **125.000** |

**Inventive examples**

**Example BIL/FON1 (direct compression process):**

| S.No. | Ingredient | mg/tab | |
|---|---|---|---|
| | **Stage-A (Sifting & Blending)** | **Function** | **mg/tab** |
| 1 | Bilastine, Form 3 (*In house*) | API | 20.072 |
| 2 | Lactose Monohydrate (Tablettose® 100) | Water-soluble filler | 72.170 |
| 3 | Cellulose, Microcrystalline *Ph. Eur.* (Avicel® PH 200) | Water-insoluble filler | 30.858 |
| 4 | Sodium Starch Glycolate *Ph. Eur.* (Explotab®) | Disintegrant | 1.000 |
| 5 | Silica, colloidal anhydrous *Ph. Eur.* (Aerosil® 200) | Glidant | 0.960 |
| | **Stage-B (Lubrication)** | | |
| 1 | Magnesium Stearate *Ph. Eur.* | Lubricant | 1.940 |
| | **Tablet Weight (mg):** | | **127.000** |

**Example BIL/FON2 (dry granulation process):**

| S.No. | Ingredient | mg/tab | |
|---|---|---|---|
| | **Stage-A (Blending & Dry Granulating)** | **Function** | **mg/tab** |
| 1 | Bilastine, Form 3 (*In house*) | API | 20.05 |
| 2 | Mannitol *Ph. Eur.* (Pearlitol® 200 SD) | Water-soluble filler | 76.50 |
| 3 | Cellulose, Microcrystalline *Ph. Eur.* (Avicel® PH 200) | Water-insoluble filler | 17.95 |
| 4 | Sodium Starch Glycolate *Ph. Eur.* (Explotab®) | Disintegrant | 5.00 |
| 5 | Silica, colloidal anhydrous *Ph. Eur.* (Aerosil® 200 Pharma) | Glidant | 2.50 |

(continued)

| S.No. | Ingredient | mg/tab | |
|---|---|---|---|
| | **Stage-A (Blending & Dry Granulating)** | **Function** | **mg/tab** |
| 6 | Magnesium Stearate *Ph. Eur.* | Lubricant | 1.00 |
| | **Stage-B (Lubrication)** | | |
| 1 | Silica, colloidal anhydrous *Ph. Eur.* (Aerosil® 200 Pharma) | Glidant | 1.00 |
| 2 | Magnesium Stearate *Ph. Eur.* | Lubricant | 1.00 |
| | **Tablet Weight (mg):** | | **125.00** |

**Example BIL/F3) (dry granulation process):**

| S.No. | Ingredient | mg/tab | |
|---|---|---|---|
| | **Stage-A (Blending & Granulating)** | **Function** | **mg/tab** |
| 1 | Bilastine, Form 3 (*In house*) | API | 20.09 |
| 2 | Mannitol *Ph. Eur.* (Pearlitol® 200 SD) | Water-soluble filler | 73.00 |
| 3 | Cellulose, Microcrystalline *Ph. Eur.* (Avicel® PH 200) | Water-insoluble filler | 24.91 |
| 4 | Sodium Starch Glycolate *Ph. Eur.* (Explotab®) | Disintegrant | 1.50 |
| 5 | Silica, colloidal anhydrous *Ph. Eur.* (Aerosil® 200) | Glidant | 0.50 |
| 6 | Magnesium Aluminometasilicate *Ph. Eur.* (Neusilin® US2) | Glidant | 2.50 |
| 7 | Magnesium Stearate *Ph. Eur.* (Valtris) | Lubricant | 1.00 |
| | **Stage-B (Lubrication)** | | |
| 1 | Silica, colloidal anhydrous *Ph. Eur.* (Aerosil® 200 Pharma) | Glidant | 0.50 |
| 2 | Magnesium Stearate *Ph. Eur.* (Valtris) | Lubricant | 1.00 |
| | **Tablet Weight (mg):** | | **125.00** |

**Example BIL/F4 (dry granulation process):**

The composition BIL/F4 is obtained exactly as example BIL/F3, but using API micronized by jet-mill. The unmicronized API ("as is" from the synthesis) was used in the previous example BIL/F3.

The particle size distribution $D_{90}$ (by volume, Malvern) of API is 8.14 micron in example BIL/F4, and 35.8 micron in BIL/F3.

**Example BIL/F5 (dry granulation process):**

| S.No. | Ingredient | mg/tab | |
|---|---|---|---|
| | **Stage-A (Blending & Granulating)** | **Function** | **mg/tab** |
| 1 | Bilastine, Form 3 (*In house*) | API | 20.05 |
| 2 | Mannitol *Ph. Eur.* (Pearlitol® 200 SD) | Water-soluble filler | 93.00 |
| 3 | Cellulose, Microcrystalline *Ph. Eur.* (Avicel® PH 200) | Water-insoluble filler | 4.45 |
| 4 | Sodium Starch Glycolate *Ph. Eur.* (Explotab®) | Disintegrant | 2.00 |

(continued)

| S.No. | Ingredient | mg/tab | |
|---|---|---|---|
| | **Stage-A (Blending & Granulating)** | **Function** | **mg/tab** |
| 5 | Silica, colloidal anhydrous *Ph. Eur.* (Aerosil® 200) | Glidant | 0.50 |
| 6 | Magnesium Aluminometasilicate *Ph. Eur.* (Neusilin® US2) | Glidant | 2.50 |
| 7 | Magnesium Stearate *Ph. Eur.* (Valtris) | Lubricant | 1.00 |
| | **Stage-B (Lubrication)** | | |
| 1 | Silica, colloidal anhydrous *Ph. Eur.* (Aerosil® 200 Pharma) | Glidant | 0.50 |
| 2 | Magnesium Stearate *Ph. Eur.* (Valtris) | Lubricant | 1.00 |
| | **Tablet Weight (mg):** | | **125.00** |

**Example BIL/F6 (dry granulation process):**

| S.No. | Ingredient | mg/tab | |
|---|---|---|---|
| | **Stage-A (Blending & Dry Granulating)** | **Function** | **mg/tab** |
| 1 | Bilastine, Form 3 (*In house*) | API | 20.05 |
| 2 | Mannitol *Ph. Eur.* (Pearlitol® 200 SD) | Water-soluble filler | 76.50 |
| 3 | Cellulose, Microcrystalline *Ph. Eur.* (Avicel® PH 200) | Water-insoluble filler | 17.95 |
| 4 | Sodium Starch Glycolate *Ph. Eur.* (Explotab®) | Disintegrant | 5.00 |
| 5 | Magnesium Aluminometasilicate *Ph. Eur.* (Neusilin® US2) | Glidant | 2.50 |
| 6 | Magnesium Stearate *Ph. Eur.* | Lubricant | 1.00 |
| | **Stage-B (Lubrication)** | | |
| 1 | Silica, colloidal anhydrous *Ph. Eur.* (Aerosil® 200 Pharma) | Glidant | 1.00 |
| 2 | Magnesium Stearate *Ph. Eur.* | Lubricant | 1.00 |
| | **Tablet Weight (mg):** | | **125.00** |

**Physical parameters of the tablets:**

| S. No. | Parameter | BIL/F3 | BIL/F4 | BIL/F5 | BIL/F6 | BITOSEN® 20mg Tabletten |
|---|---|---|---|---|---|---|
| **1** | **Average weight (mg)** | 126.10 | 125.90 | 125.90 | 125.40 | 124.5 |
| **2** | **Thickness (mm)** | 3.33-3.39 | 3.36-3.44 | 3.31-3.42 | 3.25-3.32 | 3.21-3.24 |
| **3** | **Hardness (N)** | 76-101 | 79-99 | 82-104 | 88-103 | 135-142 |
| **4** | **Disintegration time (min’ sec")** | 1’09"-1’45" | 1’18"-2’28" | 1’09"-1’50" | 55"-1’25" | 14"-20" |

## Part C

## Disintegration Study:

The disintegration time of tablets was determined according to the US Pharmacopeia (USP) test for uncoated

tablets in 37°C deionized water. The reported result is an average of 6 measurements. As can be determined from the data below, the disintegration time is very short for Bitosen® and comparative examples (less than 20 sec), and short for all other examples (less than 2.5 minutes).

| Batch | Disintegration time (sec) |
|---|---|
| Bitosen® 20 mg (B. No. 62033) | 14-20 |
| BIL/CE1 | 7-10 |
| BIL/CE2 | 12-20 |
| BIL/CE3 | 5-9 |
| BIL/CE4 | 13-20 |
| BIL/CX | 36-64 |
| BIL/FON1 | 21-30 |
| BIL/FON2 | 58-68 |
| BIL/F3 | 69-105 |
| BIL/F4 | 78-148 |
| BIL/F5 | 69-110 |
| BIL/F6 | 55-85 |

**Part D**

**Dissolution Study:**

For the tablet formulations of inventive examples and comparative examples, the dissolution test was carried out according to the following dissolution test methods and conditions.

Dissolution conditions and methods:

The test in QC release media was carried out using tablets at a paddle speed of 50 revolutions per minute (RPM) according to method 2 (Paddle) of dissolution test of the USP, using 900 mL of acetate buffer at pH 4.5. The temperature of the medium is maintained at 37°C ± 0.5°C using a water bath. Sample solutions were obtained at 5, 10, 15, 20, 30, 45 and 60 minutes after starting the test, and filtered through a 0.45 μm PVDF Millipore syringe filter.

Analogously, the dissolution is tested under the biorelevant conditions: 250 mL of acetate buffer at pH 4.5, method 2 (Paddle) with PEAK vessels at a paddle speed of 30 RPM.

The 2$^{nd}$ biorelevant conditions (used mainly for evaluation of compositions with form 2) are the same as above, but with the paddle speed of 25 RPM instead of 30 RPM.

Dissolution analytical test method details:

**Equipment:**

- A High Performance Liquid Chromatography system with isocratic elution capability, a Spectrophotometric UV detector and an auto sampler (Waters Alliance 2695 separations module, Waters 2487 dual λ absorbance detector or equivalent).

- Data handling system (Waters Empower work station or equivalent).

- Analytical column: A stainless steel column 150 mm long, 4.6 mm internal diameter filled with octadecylsilyl silica particles as a stationary phase with size 3.5μm. (Use: Xterra RP18, 150 mm length, 4.6 mm internal diameter, 3.5μm particle size or equivalent).

- Dissolution Tester (Make: Electrolab, model TDT-08L or equivalent).

Preparation of analytical solutions:

**Buffer:** Prepare 10mm Di Potassium Hydrogen Phosphate Anhydrous. For example, transfer 1.76 gm of Di Potassium Hydrogen Phosphate Anhydrous in to beaker containing 1000 mL of Milli-Q- grade water. Adjusted pH to 6.8 with Diluted Orthophosphoric acid. Filter through 0.45μ micron or finer porosity membrane filter and degas.
**Mobile phase:** Buffer: Acetonitrile (65:35 v/v).
**Preparation of diluent:** Mix water and Acetonitrile in the ratio of 50:50 (v/v) and degas.
**Preparation of dissolution media:** Prepare acetate buffer solution pH 4.5 in purified water as mentioned in the Ph. Eur. 5.17.1 For example: Dissolve 29.9 g of Sodium acetate trihydrate and 16.6 mL of Acetic acid into a 10,000 mL beaker containing 8,000 mL of purified water. Dissolve and dilute to 10000 mL with water and mix. Adjust the pH to 4.5 if necessary, with Acetic acid or diluted Sodium hydroxide solution.
**Standard solution:** Prepare a solution containing 0.022 mg/mL of Bilastine in diluent. For example, weigh and transfer about 22 mg of Bilastine working standard into 50 mL clean, dry volumetric flask add about 10 mL of diluent and sonicate to dissolve. Further add 30mL of dissolution media and sonicate for 2 minutes. Make up the volume with Dissolution media. Dilute 5 mL to 100 mL with dissolution media. Prepare it in duplicate.
**Sample solution:** Set the parameters of instrument as mentioned above. Place one tablet each in six vessels containing the dissolution medium, which has been equilibrated at 37°C±0.5°C and start the dissolution tester. At the specified time interval withdraw sample solution from each vessel. Filter through 0.45μm syringe filter, discarding first few mL of filtrate.

HPLC chromatographic conditions:

- Column: Xterra RP18, 150mm length, 4.6mm internal diameter, 3.5μm particle size or equivalent
- Flow rate: 1.0 mL/min
- Detection: UV, 215 nm
- Injection Volume: 10 μL
- Data acquisition time: 5 minutes
- Pump mode: Isocratic
- Column temperature: 30° C

**Precautions during dissolution test**

Saturate the filter with about 10 mL of sample solution before collection of samples. Use prefilter at the end of dissolution cannula during sample collection in dissolution vessel. 0.45μm PVDF (Make: Millipore or Whatman). During the sample filtration avoid entrapment of air bubbles in to the filter. In case of dissolution profiles, use separate filter at each time point.

**Evaluation of system suitability**

Equilibrate the column and system at the initial composition for 30 minutes. Inject the dissolution media as blank into the liquid chromatographic system and record the chromatogram. Inject the STD-I solution, five times into the liquid chromatographic system and record the chromatogram. Symmetry factor should be not more than 2.0 for the Bilastine peak from the standard chromatogram. %RSD for Bilastine peak areas of five injections from STD-I should be not more than 2.0. Inject STD-II solution in duplicate into the liquid chromatographic system and record the chromatogram. Calculate the similarity factor between two standard preparations. The similarity factor between two standard preparations should be in between 0.98 to 1.02.

**Calculation of Similarity factor**

$$\text{Similarity factor} = \frac{\text{Average area of STD-I}}{\text{Average area of STD-II}} \times \frac{\text{Weight of STD-II}}{\text{Weight of STD-I}}$$

**Procedure**

Inject the sample solution into the liquid chromatography and record the chromatogram.
Retention time of Bilastine is about 2.8 minutes.

**Calculation**

$$\text{\% Labelled amount as Bilastine} = \frac{At}{As} \times \frac{Ws}{50} \times \frac{5}{100} \times \frac{900}{Lc} \times P$$

where,

At : Area of peak corresponding to Bilastine in test solution chromatogram

As : Average area of peak corresponding to Bilastine obtained from STD-I chromatograms.

Ws : Weight of Bilastine working standard used for the preparation of STD-I (mg).

Lc : Label claim of Bilastine (mg).

P : % Potency of Bilastine working standard on as is basis.

**Table: Comparative dissolution profiles of Bilastine tablets 20 mg with Bitosen® 20 mg in QC release media (Acetate buffer pH 4.5, Paddle-50 RPM, 900mL)**

| Product /Batch No. | Mean Cumulative % Labelled Amount Dissolved | | | | | | |
|---|---|---|---|---|---|---|---|
| | 5 min | 10 min | 15 min | 20 min | 30 min | 45 min | 60 min |
| BITOSEN® 20mg Tabletten (B.No. 62033) | 56 | 77 | 86 | 90 | 94 | 96 | 97 |
| BIL/CE1 | 62 | 69 | 72 | 74 | 76 | 79 | 82 |
| BIL/CE2 | 51 | 62 | 67 | 72 | 75 | 78 | 80 |
| BIL/CE3 | 65 | 68 | 71 | 73 | 75 | 75 | 76 |
| BIL/CE4 | 66 | 77 | 80 | 83 | 84 | 86 | 87 |
| BIL/FON2 | 83 | 96 | 98 | 100 | 100 | 101 | 101 |
| BIL/F3 | 52 | 77 | 90 | 94 | 96 | 99 | 99 |
| BIL/F4 | 51 | 72 | 86 | 92 | 95 | 96 | 96 |
| BIL/F5 | 58 | 82 | 92 | 95 | 97 | 97 | 98 |
| BIL/F6 | 89 | 96 | 97 | 98 | 98 | 98 | 98 |

Dissolution of the inventive formulations of the present invention in QC release media (acetate buffer pH 4.5, Paddle-50 RPM, 900mL) is comparable to the commercially available reference product (Bitosen ® 20 mg Tabletten). The micronisation of Bilastine form 3 API does not improve the dissolution; it does not have any significant influence on the dissolution profiles, as evident from the comparison of the dissolution data of examples BIL/F3 and BIL/F4 having same quantitative composition, but different PSD of used API.

The dissolution for the comparative examples BIL/CE1 and BIL/CE2 is slower compared with the commercially available reference product (Bitosen ® 20 mg Tabletten), and the complete drug release is not achieved even at 60 min time point (82% BIL/CE1 and 80% for BIL/CE2 vs. 97% for Bitosen® batch 62033).

As can be seen from the table above, the dissolution profile for the comparative example BIL/CE4 is not comparable to the reference product (Bitosen ® 20 mg Tabletten) and to the inventive examples, as the complete drug release in QC media is not achieved even at 60 min time point (87% for BIL/CE4 vs. 97% for Bitosen® batch 62033). The differences of profiles in the biorelevant media are even more, as shown in table below. The examples according to the present invention show comparable dissolution profiles to the commercially available reference product (Bitosen® 20 mg) when assessed in acetate buffer. As such, it can be drawn from these data that the compositions of the present invention, as defined by the additional presence of a water-soluble filler, show improved solubility over alternative formulations (such as Example 10D of WO 2017/017301) comprising polymorph form 2 of Bilastine. Also, the exchange of polymorph 2 by polymorph 3 does not lead to an improvement, as evident from the dissolution profile of BIL/CE3 (in fact, the drug release is even lower compared with BIL/CE4). The complete drug release from composition BIL/CE3 in QC media is not achieved even at 60 min time point (76% for BIL/CE3 vs. 97% for Bitosen® batch 62033).

**Table: Dissolution profiles of Bilastine tablets 20 mg with Bitosen® 20 mg in biorelevant media (acetate buffer pH 4.5, Paddle with peak vessel - 30 RPM, 250mL) along with IVIVC predictions**

| **Product /Batch No.** | **Mean Cumulative % Labelled Amount Dissolved after time (in minutes)** | | | | | | | ***Predicted Ratio (%)*** | |
|---|---|---|---|---|---|---|---|---|---|
| | **5** | **10** | **15** | **20** | **30** | **45** | **60** | ***Cmax*** | ***AUC Inf*** |
| **BITOSEN® 20mg Tabletten (B.No. 62033)** | 22 | 37 | 49 | 59 | 73 | 85 | 92 | - | - |
| **BIL/CE1** | 20 | 27 | 31 | 34 | 40 | 47 | 52 | ***56.2*** | ***56.1*** |
| **BIL/CE2** | 20 | 27 | 32 | 36 | 42 | 50 | 56 | ***60.8*** | ***60.6*** |
| **BIL/CE3** | 16 | 19 | 21 | 23 | 26 | 30 | 33 | ***35.4*** | ***35.5*** |
| **BIL/FON2** | 31 | 50 | 62 | 69 | 77 | 84 | 87 | ***97.5*** | ***94.0*** |
| **BIL/F5** | 22 | 43 | 63 | 77 | 87 | 91 | 94 | ***106.5*** | ***102.3*** |
| **BIL/F6** | 42 | 59 | 71 | 77 | 84 | 89 | 91 | ***102.7*** | ***97.9*** |

Dissolution of the inventive formulations of the present invention in biorelevant media (acetate buffer pH 4.5, Paddle with peak vessel - 30 RPM, 250mL) is comparable to the commercially available reference product (Bitosen ® 20 mg Tabletten), as is required in order to achieve a bioequivalent product to Bitosen ®.

The comparability with the reference product is also evident when shown with In-vitro In-vivo correlation (IVIVC) prediction, calculated with WinNonlin software based on dissolution profiles in the biorelevant media. Based on IVIVC calculation for composition BIL/FON2, the predicted Cmax is 97.5%, and predicted AUC Inf is 94%, being in the regulatory needed interval of 80-125% for Cmax and AUC. For composition BIL/F5, the predicted Cmax is 106.5%, and predicted AUC Inf is 102.3%, being in the regulatory needed interval of 80-125% for Cmax and AUC. For composition BIL/F6, the predicted Cmax is 102.7%, and predicted AUC Inf is 97.9%, being in the regulatory needed interval of 80-125% for Cmax and AUC.

As can be seen from the table above, the dissolution profile for the comparative examples BIL/CE1 and BIL/CE2 are not comparable to the commercially available reference product (Bitosen ® 20 mg Tabletten) and to the inventive examples, as the drug release in the biorelevant media is significantly lower at 60 min time point for test product compared with the reference product (52% for BIL/CE1 and 56% for BIL/CE2 vs. 92% for Bitosen® batch 62033).

The missing comparability of BIL/CE1 and BIL/CE2 with the reference product Bitosen® is also evident from the IVIVC prediction: the predicted Cmax is 56.2-60.8%%, and predicted AUC is 56.1- 60.6%, being significantly below the regulatory needed interval of 80-125% for Cmax and AUC.

The dissolution profile for the comparative example BIL/CE3 is also not comparable to the reference product

(Bitosen ® 20 mg Tabletten) and to the inventive examples, as the drug release in the biorelevant media is significantly lower at 60 min time point for test product compared with the reference product (33% for BIL/CE3 vs. 92% for Bitosen® batch 62033). The missing comparability of BIL/CE3 with the reference product Bitosen® is also evident from the IVIVC prediction: the predicted Cmax is 35.4%, and predicted AUC is 35.5%, being significantly below the regulatory needed interval of 80-125% for Cmax and AUC.

**Table: Dissolution profiles of BIL/CE4 with Bitosen® 20 mg in 2nd biorelevant media (acetate buffer pH 4.5, Paddle with peak vessel - 25 RPM, 250mL) along with IVIVC predictions**

| **Product /Batch No.** | **Mean Cumulative % Labelled Amount Dissolved after time (in minutes)** | | | | | | | ***Predicted Ratio (%)*** | |
|---|---|---|---|---|---|---|---|---|---|
| | **5** | **10** | **15** | **20** | **30** | **45** | **60** | ***Cmax*** | ***AUC Inf*** |
| **BITOSEN® 20mg Tabletten (B.No. 53047)** | 19 | 32 | 43 | 53 | 66 | 78 | 86 | - | - |
| **BIL/CE4** | 12 | 18 | 23 | 27 | 35 | 42 | 49 | ***57.6*** | ***56.8*** |

The dissolution profile of the composition BIL/CE4 according to Example 10D of WO 2017/017301 is slower than the reference product Bitosen® and is not acceptable in the 2nd biorelevant media. The same drug release is not achieved even at 60 min time point (49% vs. 86% for Bitosen). The similarity factor (f2 value) is 29. As the f2 value is below 50, the test product is not identical in dissolution with the reference product. The missing comparability of BIL/CE4 with the reference product Bitosen® is also evident from the IVIVC prediction. For composition BIL/CE4, the predicted Cmax is 57.6%, and predicted AUC Inf is 56.8%, being significantly below the regulatory needed interval of 80-125% for Cmax and AUC.

As can be seen from the tables above, the inventive compositions are improved with respect to their dissolution times compared to the comparative examples when assessed using acetate buffer at pH 4.5. The presence of a water-soluble filler therefore enables the improvement of dissolution of polymorph 3, such that it is comparable to the composition comprising polymorph 1. Dissolution of the inventive formulations of the present invention in Acetate buffer at pH 4.5 is comparable to the commercially available reference product (Bitosen ® 20 mg Tabletten).

**Part E**

**Blend Uniformity and Content Uniformity of Tablets:**

To ensure the consistency of dosage units, each unit in a batch should have an active substance content within a narrow range around the label claim. Dosage units are defined as dosage forms containing a single dose or a part of a dose of an active substance in each dosage unit. The term "Uniformity of dosage unit" is defined as the degree of uniformity in the amount of the active substance among dosage units. Therefore, the requirements of this regulation can be applied to any active substance being comprised in dosage units containing one or more active substances. The uniformity of dosage forms (by content uniformity) was assessed according to European Pharmacopeia 2.9.40. The assay (%) of 10 individual samples (tablets or locations in blend) was detected by HPLC analysis according to the following HPLC conditions:

**Instrumentation**

- A High Performance Liquid Chromatography system with isocratic elution capability, a Spectrophotometric UV detector and an auto sampler (Waters Alliance 2695 separations module, Waters 2489 dual λ absorbance detector or equivalent).

- Data handling system (Waters Empower work station or equivalent).

- Analytical column: A stainless steel column 150 mm long, 4.6 mm internal diameter filled with octadecylsilyl silica particles as a stationary phase with size 3.5 μm. (Use: XBridge Shield RP18, 150 mm length, 4.6 mm internal diameter, 3.5μm particle size or equivalent.)

**Reagents**

- Water: HPLC / Milli-Q- grade
- Acetonitrile: HPLC grade (Make: Merck)
- Di Potassium hydrogen Phosphate: Emparta grade (Make: Merck)
- Ortho phosphoric acid: AR grade (Make: Merck)

*Note: All the reagents/solvents can be used above mentioned make or equivalent*

**Preparation of Mobile phase**

- **Buffer:** Prepare 10mm Di Potassium Hydrogen Phosphate Anhydrous. For example, transfer 1.76 gm of Di Potassium Hydrogen Phosphate Anhydrous in to beaker containing 1000 mL of Milli-Q- grade water. Adjusted pH to 6.8 with Diluted Orthophosphoric acid Filter through 0.45μ micron or finer porosity membrane filter and degas.
- **Mobile phase:** Buffer: Acetonitrile (70:30 V/v).
- **Preparation of diluent:** Mix water and Acetonitrile in the ratio of 50:50 (v/v) and degas.

**Chromatographic conditions**

- Column : XBridge Shield RP18, 150mm length, 4.6mm internal diameter, 3.5μm particle size or equivalent.
- Flow rate: 1.0 mL/min
- Detection UV, 215 nm
- Injection Volume: 10 μL
- Data acquisition time: 8 minutes
- Column temperature: 30°C
- Pump mode: Isocratic

**Preparation of solutions**

**Standard solution:** Prepare a solution containing 0.08 mg/mL of Bilastine in diluent.

For example, weigh accurately 50 mg of Bilastine working standard into a 50 mL clean, dry volumetric flask. Add 15 mL of diluent and sonicate to dissolve. Make up to volume with diluent and mix. Dilute 4.0 mL of this solution to 50 mL with diluent. Label this working standard solution as the STD-I. Prepare the working standard solution in duplicate and label it as STD-II.

**Sample solution:** For Example: Carefully transfer entire amount of sample from glass vial into a 200 mL volumetric flask by using funnel. Rinse the vial completely with water and transfer the rinsing volume into the same volumetric flask. Repeat the rinsing of vial procedure at least 3 times so that entire sample will transfer into volumetric flask. Add 50 mL diluent shake for 20 minutes at 150 RPM. Add 50 mL diluent and sonicate for 15 minutes with intermittent shaking, make up the volume with diluent. Shake well and filter through 0.45 micron membrane filter. Further dilute 5 mL to 20 mL with diluent.

**Evaluation of system suitability**

- Equilibrate the column and system at the initial composition for 30 minutes.
- Inject the diluent as blank into the liquid chromatographic system and record the chromatogram.
- Inject the STD-I solution, five times into the liquid chromatographic system and record the chromatogram.
- Symmetry factor should be not more than 2.0 for the Bilastine peak from the standard chromatogram. Retention time of Bilastine peak is about 4.7 minutes.
- %RSD for Bilastine peak areas of five injections from STD-I should be not more than 2.0.
- Inject STD-II solution in duplicate into the liquid chromatographic system and record the chromatogram.
- Calculate the similarity factor between two standard preparations. The similarity factor between two standard preparations should be in between 0.98 to 1.02.

**Calculation of Similarity factor**

$$\text{Similarity factor} = \frac{\text{Average area of STD-I}}{\text{Average area of STD-II}} \times \frac{\text{Weight of STD-II}}{\text{Weight of STD-I}}$$

**Calculation**

$$\text{Assay (\% Label claim)} = \frac{At}{As} \times \frac{Ws}{50} \times \frac{4}{50} \times \frac{200}{Wt} \times \frac{20}{5} \times \frac{LQ}{LC} \times P$$

Where,

At : Area of peak corresponding to Bilastine in test solution chromatogram
As : Average area of peak corresponding to Bilastine obtained from STD-I
Ws : Weight of Bilastine standard used for the preparation of STD-I (mg).
Wt : Weight of sample (mg)
LC : Label claim of Bilastine (mg)
P : % Assay of Bilastine working standard on as is basis.
LQ : Label Quantity of Bilastine (mg)

The results are presented in the table below.

**Table: Results from assessment of content uniformity and assay of tablets batch BIL/F6**

| Uniformity of dosage units | BIL/F21 |
|---|---|
| Tablet-1 | 101.1 |
| Tablet-2 | 101.4 |
| Tablet-3 | 102.8 |
| Tablet-4 | 101.4 |
| Tablet-5 | 102.1 |
| Tablet-6 | 103.7 |
| Tablet-7 | 100.3 |
| Tablet-8 | 103.2 |
| Tablet-9 | 104.2 |
| Tablet-10 | 103.1 |
| **Mean** | **102.3** |
| **%RSD** | **1.24** |
| **Assay** | **101.5** |

As can be observed from the data presented above, the content uniformity is found to be within the specification limits according to European Pharmacopeia 2.9.40. Furthermore, both the assay of tablets and the average amount of API contained per tablet (as described above under "Mean") demonstrate a value of 101.5% and 102.3%, correspondingly, clearly satisfying the standard requirements of 95-105%.

**Part F**

**Stability Studies:**

Bilastine 20 mg Tablets were loaded into the below mentioned conditions and tablets were analyzed for Dissolution at respective time point.

**Table: Dissolution profiles of the formulation BIL/CX with form 2 (Initial & 1 month at 40°C/75% RH in Alu-Alu blister) compared with Innovator in QC release media (Acetate buffer pH 4.5, Paddle, 50 RPM, 900mL)**

| Product /Batch No. | Mean Cumulative % Labelled Amount Dissolved | | | | | | | |
|---|---|---|---|---|---|---|---|---|
| | 5 min | 10 min | 15 min | 20 min | 30 min | 45 min | 60 min | *F2* |
| BITOSEN® 20mg Tabletten (B.No. 62033) | 56 | 77 | 86 | 90 | 94 | 96 | 97 | - |

(continued)

| Product /Batch No. | Mean Cumulative % Labelled Amount Dissolved | | | | | | | |
|---|---|---|---|---|---|---|---|---|
| | **5 min** | **10 min** | **15 min** | **20 min** | **30 min** | **45 min** | **60 min** | ***F2*** |
| BIL/CX (Initial) | 67 | 84 | 90 | 93 | 96 | 99 | 99 | *Not applicable* |
| BIL/CX (1 Month at 40°C/ 75%RH in Alu-Alu blister) | 48 | 71 | 79 | 82 | 91 | 94 | 97 | 59 |

As can be observed from the table above, the dissolution profile of the formulation BIL/CX with form 2 shows a slight drop in the dissolution profile at the 5-20 minute time point of the experiment, seen after the 1 month storage in Alu-Alu blister under ICH accelerated conditions (40°C/75% RH).

Inventive Bilastine 20 mg Tablets were loaded into the below mentioned conditions and tablets were analyzed in the corresponding tests (Dissolution, Related Substance, and Assay) at respective time point.

**Table: Stability data of BIL/F6 at ICH Accelerated condition (40°C/75 % RH, Alu - Alu blister pack)**

| Test | Specification | | Initial | 1 month |
|---|---|---|---|---|
| **Dissolution (% Labelled amount, by HPLC)** | Not less than 75% (Q) of the labeled amount of Bilastine is dissolved in 30 minutes. | 5 min | 89 | 87 |
| | | 10 min | 96 | 92 |
| | | 15 min | 97 | 94 |
| | | 20 min | 98 | 95 |
| | | 30 min | 98 | 95 |
| | | 45 min | 98 | 96 |
| | | 60 min | 98 | 97 |
| **Related Substances (per cent, by HPLC)** | | | | |
| **a) Hydroxy Impurity** | Not more than 0.15 | | 0.07 | 0.06 |
| **b) Acid Impurity** | Not more than 0.15 | | ND | ND |
| **c) Di-acid Impurity** | Not more than 0.15 | | 0.02 | 0.03 |
| **d) Unspecified impurity** | Not more than 0.15 | | 0.04 | 0.03 |
| **e) Total impurities** | Not more than 2.0 | | 0.12 | 0.12 |
| **Assay (% Label claim, by HPLC)** | 95.0 -105.0 | | 101.5 | 97.5 |

As can be observed from the table above, the dissolution profile of the test formulation BIL/F6 with addition of Mannitol shows comparable dissolution profile after the 1-month storage of the tablets under ICH accelerated conditions (40°C/75% RH in Alu-Alu blister), when compared to the fresh tablets. No changes are observed (like a drop in the dissolution profile at the 5-20-minute time frame of the experiment). There is also no significant influence of temperature and humidity observed on Related Substances of Bilastine. Furthermore, the Assay of tablets demonstrates a value of 101.5% (initial) and 97.5% (after 1 month), correspondingly, clearly satisfying the standard requirements of 95-105%.

**Table: Stability data of BIL/F6 at ICH Long term condition (25°C/60%RH, Alu - Alu blister pack)**

| Test | Specification | | Initial | 1 month |
|---|---|---|---|---|
| Dissolution (% Labelled amount, by HPLC) | Not less than 75% (Q) of the labeled amount of Bilastine is dissolved in 30 minutes. | 5 min | 89 | 88 |
| | | 10 min | 96 | 93 |
| | | 15 min | 97 | 95 |
| | | 20 min | 98 | 96 |
| | | 30 min | 98 | 97 |
| | | 45 min | 98 | 97 |
| | | 60 min | 98 | 99 |
| Related Substances (per cent, by HPLC) | | | | |
| a) Hydroxy Impurity | Not more than 0.15 | | 0.07 | 0.07 |
| b) Acid Impurity | Not more than 0.15 | | 0.02 | 0.03 |
| c) Di-acid Impurity | Not more than 0.15 | | ND | ND |
| d) Unspecified impurity | Not more than 0.15 | | 0.04 | 0.02 |
| e) Total impurities | Not more than 2.0 | | 0.12 | 0.12 |
| Assay (% Label claim, by HPLC) | 95.0 -105.0 | | 101.5 | 97.5 |

As is evident from the table above, there is no significant influence of temperature and humidity observed on Dissolution and Related Substances of Bilastine in selected composition of BIL/F6 after the storage under ICH long term conditions for 1 month in Alu-Alu blister. Furthermore, the Assay of tablets demonstrates a value of 101.5% (initial) and 97.5% (after 1-month long term storage), correspondingly, clearly satisfying the standard requirements of 95-105%.

**Table: Related Substances data at ASAP conditions for BIL/F6 under open exposure**

| S. No. | Condition | Days | Hydroxy Impurity | Acid Impurity | Di-Acid Impurity | Maximum Unknown (0.71 RRT) | Total impurities |
|---|---|---|---|---|---|---|---|
| 1 | Initial | -- | 0.07 | ND | 0.02 | 0.04 | 0.12 |
| 2 | 50°C/ 75%RH | 14 | 0.07 | ND | 0.02 | 0.03 | 0.18 |
| 3 | 60°C/ 40%RH | 14 | 0.07 | ND | 0.02 | 0.03 | 0.17 |
| 4 | 60°C/ 75%RH | 14 | 0.07 | ND | 0.02 | 0.03 | 0.18 |
| 5 | 70°C/ 5%RH | 14 | 0.07 | ND | 0.03 | 0.03 | 0.18 |
| 6 | 70°C/ 75%RH | 2 | 0.06 | ND | 0.03 | 0.03 | 0.19 |
| 7 | 80°C/ 40%RH | 2 | 0.07 | ND | 0.03 | 0.03 | 0.21 |

As is evident from the table above, related substances data at all the ASAP conditions under open exposure were found to be satisfactory as total impurity levels are low (not more than 2% m/m).

## Part G

**Polymorphic Analysis of API:**

The XRD analysis of API before and after micronisation is confirming that API remains in the polymorphic form 3 without changes during micronisation process.

The corresponding powder X-ray diffraction results are demonstrated in the figures below (Figures 3 and 4).

During the earlier trials of micronisation of pure polymorphic form 2, the presence of form 3 and dihydrate form A as polymorphic impurities is observed in some cases after jet milling. For example, two from five batches of micronized API have shown the contamination of form 2 with form 3 and dihydrate form A.

**Polymorphic Stability of Tablets:**

Additional analysis has also demonstrated a high stability of the polymorphic form 3 in the compositions of the present invention. The assessment of polymorphic stability of the API in tablets produced as described above was carried out using the powder X-ray diffraction (XRD) analysis of fresh and stored tablets.

The physical (polymorphic) and chemical stability of the inventive pharmaceutical tablet composition may be tested in a conventional manner, in particular under long term conditions (25°C/60 % RH in commercial blister packaging) and under accelerated conditions (at 40°C/75 % RH in commercial blister packaging) according to ICH guideline Q1A(R2). A 2-week Accelerated Stability Assessment Program (ASAP), according to Waterman 2011 (Waterman KC, The application of the Accelerated Stability Assessment Program (ASAP) to quality by design (QbD) for drug oroduct stability, AAPS PharmSciTech, Vol. 12, No. 3, September 2011) is usually applied for a faster estimation of chemical stability and for a more precise shelf-life prediction compared with ICH stability. Analogously to the chemical stability, an ASAP study can be applied for the quick estimation of the physical stability of a composition or a precise relative comparison of polymorphic stability of two different compositions.

Compositions according to BIL/F3 and BIL/F6 were assessed by powder XRD at initial timepoint and after storage (open exposure or in Alu-Alu blister) at various temperature and humidity conditions, for example:

1) after ICH stability study under long term conditions (25°C/60 % RH in Alu-Alu blister),
2) after ICH stability study under accelerated conditions (40°C/75%RH in Alu-Alu blister),
3) after ASAP study under stress conditions (e.g., open exposure at 50°C/75%RH 14 Days).

The results are presented in the table below.

**Table: Polymorphic stability studies overview**

| S.No | Batch No | Condition | API polymorphic form identification by XRD |
|---|---|---|---|
| 1 | BIL/CX | Initial | Form 2 |
| 1 | BIL/CX | 40°C/75%RH, 1 month in Alu-Alu blister | Form 2 |
| 1 | BIL/CX | 50°C/75%RH 14 Days open exposu re | Form 2 with traces of Dihydrate form A |
| 1 | BIL/F3 | Initial | Form 3 |
| 2 | BIL/F3 | 40°C/75%RH, 1 month in Alu-Alu blister | Form 3 |
| 3 | BIL/F6 | Initial | Form 3 |
| 4 | BIL/F6 | 25°C/60%RH, 2 months in Alu-Alu blister | Form 3 |
| 4 | BIL/F6 | 40°C/75%RH, 2 months in Alu-Alu blister | Form 3 |
| 5 | BIL/F6 | 50°C/75%RH 14 Days open exposu re | Form 3 |

As shown in the table above, the powder X-ray diffraction analysis of the API in tablets produced as described above according to BIL/F3 shows that only polymorphic form 3 is present at initial time point and also after 1 month storage under ICH accelerated conditions (40°C/75%RH, 1 month in Alu-Alu blister).

The accelerated stability test as set out above have been performed according to applicable pharmaceutical regulatory standards as described in ICH guideline Q1A(R2), wherein the pharmaceutical tablet composition have been packed in Alu-Alu blisters.

The bilastine API used in the tablet composition BIL/CX is the polymorphic form 2. The amount of polymorphic

impurity (Dihydrate form A) measured for the composition initially after production is below detection limit, and only form 2 of Bilastine was detected. When using the regulatory accelerated stability assessment standards as storage conditions, the tablet composition is sealed in Alu-Alu blisters and the amount of polymorphic impurity (Dihydrate form A) is still below detection limit after 1 month storage. Accordingly, the pharmaceutical tablet compositions according to example BIL/CX shows no physical instability (polymorphic transformation) within one month accelerated storage.

For the composition BIL/CX, the contamination with traces of Dihydrate form A is observed in ASAP study after the open exposure storage at high temperature/high humidity (50°C/75%RH) for 14 days. This polymorphic impurity was found only at this high temperature/high humidity conditions and only after the maximal storage time in the ASAP study (2 weeks).

At lower humidity, this polymorphic impurity has not been detected even at higher temperatures (e.g. 70°C/5%RH 14 Days). At shorter storage time, this polymorphic impurity has also not been detected even at higher temperatures (e.g. 70°C/75%RH 2 Days).

The powder X-ray diffraction analysis of the API in tablets produced as described above according to inventive example BIL/F6 shows that only polymorphic form 3 is present at initial time point and after 14 days of stress storage at high temperature/high humidity (50°C/75%RH, open exposure). The ASAP data as set out above already suggests that tablets BIL/F6 packaged in commercial Alu-Alu blister will show no Dihydrate form A impurity after storage under ICH accelerated and long term conditions, as the water vapor transmission rate (WVTR) for Alu-Alu blister is reduced to the minimum. The finding is confirmed by XRD results after 2 months storage in Alu-Alu blister under ICH accelerated conditions (40°C/75%RH) and long term conditions (25°C/60%RH).

For a precise comparison of physical (polymorphic) stability of two compositions BIL/CX (with form 2) and BIL/F6 (with form 3), the XRD results after most stressing conditions in the ASAP study are more suitable. Unlike in the composition BIL/CX, no traces of polymorphic impurity of Dihydrate form A are detected in tablets according to BIL/F6 after 14 days of open exposure storage at high temperature/high humidity (50°C/75%RH) showing that the composition BIL/F6 is more polymorphic stable than BIL/CX. Accordingly, higher shelf-life can be expected for the pharmaceutical tablets according to example BIL/F8 in same packaging material (Alu-Alu) or a much cheaper blister with higher water vapor transmission rate, like PVC blister, could be used as a commercial primary packaging.

The above stability results for tablets are unexpected in view of the prior art, as WO 2017/017301 is disclosing that form 2 of Bilastine API is stable on storage, whilst form 3 of Bilastine API is converted partially into the form 1 leading to a mixture of form 3 with form 1 after 1 month storage at 25°C/60%RH (ICH long term conditions) and at 40°C/75%RH (ICH accelerated conditions), and the phase transformation is increasing further after 2 months under accelerated conditions to afford a mixture of form 1 with form 3.

Exemplary powder X-ray diffraction results are demonstrated in the figures below (Figures 1-12).

The powder X-ray diffraction analysis of Bilastine API Form 1 (Fig. 1) and Form 2 (Fig. 2) is done according to below "XRD method D1".

Unmicronized Bilastine API Form 3 (Fig. 3) is analyzed according to below "XRD method V1".

The PXRD analysis of Bilastine micronized API Form 3 (Fig. 4) and 3 tablet samples of BIL/F6 (Figures 10-12) is done by "XRD method A1".

Dihydrate form A of Bilastine (Fig. 5) and tablet samples of BIL/CX and BIL/F3 (Figures 6-9) are analysed using the "XRD method V2".

XRD method D1:

The qualitative PXRD measurements were performed using the X'Pert MPD system, LLF x-ray source with CuKa radiation, Soller collimators of 0.02, 0.04 rad, divergent slit ½ deg, powder load was 400 mg. The radiation used - CuKa (8keV); 40kV-40mA; measurement range 0-40 degrees, measurement time per scan - 2.5 hours, step = 0.013° 2θ. During the measurements the sample was rotated at speed 4s/revolution to improve counting statistic.

XRD method V1:

A summary of XRD method parameters is described below.

| | |
|---|---|
| Model/ Make: | Bruker AXS/D8 Advance |
| X-Ray Tube: | Copper Ka1, 1.5406 A |
| Detector: | Lynx Eye |
| Kβ Filter: | Nickel |
| Scan range (° 2θ): | 3 - 40 |
| Step Size (° 2θ): | 0.016° |

(continued)

| | |
|---|---|
| Current: | 40 mA |
| Voltage: | 40 kV |
| Scan Type: | Locked coupled and continuous |
| Spinning: | 30 rpm |
| Divergence Slit: | 0.3° |
| Anti-Scattering Slit: | 3 mm |
| Scan Time: | 20 min |

*Sample preparation:*

Fill the fine powder into the round cavity of the sample holder and smooth the surface with glass plate/slide to obtain smooth surface. The sample surface should be in parallel to holder surface and free from crevices and cracks. Clean the outer edges of the holder with tissue paper to avoid sample contaminations.

Place the prepared sample holder carefully on the sample stage of the XRD instrument and analyze as per above method conditions at room temperature using the Diffrac plus software.

XRD method A1:

A summary of XRD method parameters is described below.

| | |
|---|---|
| Make/ Model | PANalytical / EMPYREAN |
| X-Ray tube | Copper Ka1 |
| Wavelength (A) | 1,5406 |
| Detector | PIXcel 1D |
| Mode of analysis | Reflection |
| Scan range (° 2θ) | 3 - 40 |
| Step size (° 2θ) | 0,013 |
| Current | 40 mA |
| Voltage | 45 kV |
| Scan type | Locked coupled, continuous |
| Spinning | 30 rpm |
| Divergence Slit | 0.25°, Fixed |
| Anti-scattering slit | 0.5°, Fixed |
| Scan time | 1 Hr |

*Sample preparation:*

*API samole:* The API powder sample is measured as is.

*Tablet samples:* Take four tablets, grind to fine powder using Agate mortar and pestle gently.

Prepare the sample (approx. 350mg) using PANalytical sample preparation kit by 'Back loading technique'*. The sample surface should be smooth and in parallel to sample holder surface. Clean the outer edges of the holder with tissue paper to avoid sample contaminations.

*Sample preparation by 'Back Loading Technique'

- Ensure the sample holder ring is securely clamped to the preparation table.
- Place the sample in the holder ring and press the sample powder down firmly using the press block.
- Remove all surplus powder above the rim of the holder ring using the knife blade and brush.
- Place the bottom plate on to the holder ring and clamp in position.
- Remove the complete sample holder from the preparation table by turning the preparation table upside down and

pressing the spring loaded knob.

XRD method V2:

A summary of XRD method parameters is described below.

| | |
|---|---|
| Model/ Make: | Bruker AXS/D8 Advance |
| X-Ray Tube: | Copper Ka1, 1.5406 A |
| Detector: | Lynx Eye |
| Kβ Filter: | Nickel |
| 2θ Start: | 3.0° |
| 2θ End: | 40.0° |
| Step Size: | 0.016° |
| Current: | 40 mA |
| Voltage: | 40 kV |
| Scan Type: | Locked coupled and continuous |
| Spinning: | 30 rpm |
| Divergence Slit: | 0.3° |
| Receiving Slit: | 3 mm |
| Total Time: | 1 Hr |

*Sample preparation:*

*API samole:* The API powder sample is measured as is.

*Tablet samples:* Take four tablets, grind to fine powder using neat and clean mortar and pestle.

Fill the fine powder in the round cavity of the sample holder and smooth the surface with glass plate/slide to obtain smooth surface. The sample surface should be in parallel to holder surface. Clean the outer edges of the holder with tissue paper to avoid sample contaminations.

Place the prepared sample holder on the sample stage of the XRD instrument and analyze as per the above method at room temperature.

**Part H**

**Comparison to Examples 2 and 4 of CN106692090A:**

This data was prepared to highlight the observations associated with trials executed according to 2 and 4 of CN106692090A (Comparative examples).

**Table: Trial with compositions according to examples 2 and 4 of CN106692090A (Comparative examples)**

| S. No. | Ingredients | As per Ex. 2 | As per Ex. 4 |
|---|---|---|---|
| | | BIL/CE5 | BIL/CE6 |
| | **Stage - A (Sifting & Blending)** | **mg/tablet** | |
| 2 | Bilastine Form-3 (*In-house*)(*D90~35.8*μ) | 20.050 | 20.050 |
| 3 | Lactose Monohydrate *Ph.Eur.* (Granulac® 200) | - | 41.000 |
| 4 | Mannitol *Ph.Eur.* (Pearlitol® 25 C) | 20.000 | - |
| 5 | Cellulose, Microcrystalline *Ph.Eur.* (Comprecel® M 101 D+) | 45.950 | 24.950 |
| 6 | Sodium starch Glycolate *Ph.Eur.* (Explotab®) | 5.000 | - |
| 7 | Low-substituted hydroxypropyl cellulose *Ph.Eur.* (L-HPC LH-21) | - | 5.000 |

(continued)

| | | | |
|---|---|---|---|
| | **Stage - B (Wet granulation/Wet Sieving)** | | |
| 1 | 30% Ethanol *Ph.Eur.* in Water, Purified *Ph.Eur.* | q.s. | q.s. |
| | **Stage - C (Lubrication)** | | |
| 1 | Sodium starch Glycolate *Ph.Eur.* (Explotab®) | 5.000 | - |
| 2 | Low-substituted hydroxypropyl cellulose *Ph.Eur.* (L-HPC LH-21) | - | 5.000 |
| 3 | Silica, colloidal anhydrous *Ph.Eur.* (Aerosil® 200 Pharma) | 1.000 | 1.500 |
| 4 | Aspartame *Ph.Eur.* | 2.000 | 2.000 |
| 5 | Magnesium Stearate *Ph. Eur.*(Valtris) | 1.000 | 0.500 |
| | ***Tablet weight (mg)*** | **100.000** | **100.000** |

**General procedure:**

1) Sift API through # 100 mesh.

2) Sift intragranular ingredients of Stage-A through # 80 mesh.

3) Load the Stage-A sifted ingredients into a Rapid mixer granulator and mixed for 10 min at slow speed with Impeller ON and Chopper OFF.

4) Granulate the Step-3 material using Binder solution of Stage- B.

5) Sieve the wet material of Step-4 using # 18 mesh sieve to get wet granules.

6) Dry the granules of step-5 material in a Retch Rapid Dryer at 50°C, 10 air flow for 1.5 hours.

7) Sift the dried granules of step-6 through # 20 mesh.

8) Sift extragranular ingredients of Stage-C except Magnesium Stearate through # 80 mesh.

9) Sift Magnesium Stearate of Stage-C through # 35 mesh (or suitable size).

10) Load the step-7 and step-8 materials into a suitable blender and lubricate them using sifted Magnesium Stearate of step-9 for 5 min at 20 rpm.

11) Compress the tablets on a rotary tablet compression machine using 6.00 mm round biconcave tooling.

**Table: Physical parameters of tablets**

| Parameter | BITOSEN® 20 mg Tabletten (53047) | BIL/CE5 | BIL/CE6 |
|---|---|---|---|
| Average Weight (mg) | 124.5 | 100.6 | 101.9 |
| Thickness (mm) | 3.21-3.24 | 3.54-3.68 | 3.38-3.42 |
| Hardness (N) | 135-142 | 46-57 | 42-64 |
| Disintegration time (sec) | 14-20 | 20-25 | 10-14 |

Disintegration time of the test batches are in line with the reference product. No sticking tendency was observed

during the compression for BIL/CE5. Sticking was observed during the compression for BIL/CE6.

**Table: P-XRD analysis of tablets.**

| S. No. | Batch No. | XRD Result |
|---|---|---|
| 1 | BIL/CE5 | The API reflexes in the diffractogram match with **Bilastine Dihydrate Form A** by XRD qualitative analysis. API polymorphic form conversion was observed i.e., Bilastine Form-3 to Dihydrate Form A. |
| 2 | BIL/CE6 | The API reflexes in the diffractogram match with **Bilastine Dihydrate Form A** by XRD qualitative analysis. API polymorphic form conversion was observed i.e., Bilastine Form-3 to Dihydrate Form A. |

As shown in the table above, qualitative analysis shows that the compositions manufactured according to examples 2 and 4 of CN106692090A using wet granulation lead to polymorphic instability, thereby showing that these methods of manufacture and compositions are not capable of providing a solution to the problem of providing a stable bilastine composition based on polymorphic form 3.

**Table: Comparative dissolution profiles of Bilastine 20 mg tablets with Bitosen® 20 mg in Acetate buffer pH 4.5, Paddle with peak vessel - 30 RPM, 250mL**

| Product /Batch No. | Mean Cumulative % Labelled Amount Dissolved over time (min) | | | | | | | | Predicted PK | | Predicted Ratio | |
|---|---|---|---|---|---|---|---|---|---|---|---|---|
| | 5 | 10 | 15 | 20 | 30 | 45 | 60 | *F2* | Cmax | AUC Inf | Cmax | AUC Inf |
| BITOSEN® 20mg Tabletten (B.No. 62033) | 22 | 37 | 49 | 59 | 73 | 85 | 92 | - | 212.7 | 913.7 | - | - |
| B. No. BIL/CE5 | 26 | 31 | 36 | 39 | 44 | 50 | 54 | ***34*** | 122.9 | 530.9 | **57.8** | **58.1** |
| B. No. BIL/CE6 | 18 | 26 | 31 | 35 | 43 | 50 | 54 | ***32*** | 124.4 | 533.6 | **58.5** | **58.4** |

**Table: Comparative dissolution profiles of Bilastine 20 mg tablets with Bitosen® 20 mg in Acetate buffer pH 4.5, Paddle - 50 RPM, 900mL**

| Product /Batch No. | Mean Cumulative % Labelled Amount Dissolved | | | | | | | |
|---|---|---|---|---|---|---|---|---|
| | 5 min | 10 min | 15 min | 20 min | 30 min | 45 min | 60 min | *F2* |
| BITOSEN® 20mg Tabletten (B.No. 62033) | 56 | 77 | 86 | 90 | 94 | 96 | 97 | - |
| B. No. BIL/CE5 | 91 | 95 | 96 | 96 | 96 | 96 | 97 | - |
| B. No. BIL/CE6 | 91 | 96 | 97 | 97 | 97 | 98 | 98 | - |

Trials executed with comparative examples according to 2-4 of CN106692090A shows polymorphic transformation from pure Form-3 (in B.No. BIL/CE5 and B.No. BIL/CE6) to 'Bilastine Dihydrate Form A' suggesting that Wet granulation is not the suitable method for development of Bilastine Tablets using form-3 API.

Dissolution profiles of the comparative examples according to 2-4 of CN106692090A shows faster dissolution profiles than reference product Bitosen® in the QC release medium. However, the dissolution profiles were considerably slow and incomplete in the biorelevant dissolution medium for all the trials executed. The IVIVC predictions of Cmax and AUC suggest that the batches BIL/CE5, BIL/CE6 are not identical or functionally similar to the reference product.

## Claims

**1.** A pharmaceutical composition in the form of a tablet, comprising a) a crystalline form 3 of bilastine, wherein the

crystalline form 3 has characteristic peaks at 6.47, 12.81, 15.70, and 17.71 ± 0.2 degrees 2-theta in a powder X-ray diffraction pattern, b) a water-soluble filler, and optionally c) a water-insoluble filler.

**2.** The pharmaceutical composition according to claim 1, wherein the composition further comprises a disintegrant, a glidant and/or a lubricant.

**3.** The pharmaceutical composition according to any one of the preceding claims, wherein the water-soluble filler is a sugar, preferably mannitol or lactose.

**4.** The pharmaceutical composition according to any one of the preceding claims, wherein the composition comprises a water-insoluble filler, which is microcrystalline cellulose.

**5.** The pharmaceutical composition according to any one of the preceding claims, wherein the composition comprises a disintegrant, selected from sodium starch glycolate, low-substituted hydroxypropylcellulose, pregelatinized starch, or crospovidone, preferably sodium starch glycolate or crospovidone.

**6.** The pharmaceutical composition according to any one of the preceding claims, wherein the composition comprises a glidant, which is silicon dioxide, preferably as colloidal anhydrous silica, magnesium aluminometasilicate and/or talc, preferably wherein the glidant is a combination of silicon dioxide and magnesium aluminometasilicate.

**7.** The pharmaceutical composition according to any one of the preceding claims, wherein the composition comprises a lubricant, which is magnesium stearate.

**8.** The pharmaceutical composition according to any one of the preceding claims, comprising a crystalline form 3 of bilastine, wherein the crystalline form 3 has characteristic peaks at 6.47, 12.81, 15.70, and 17.71 ± 0.2 degrees 2-theta in a powder X-ray diffraction pattern, a water-soluble filler, a water-insoluble filler, a disintegrant, a glidant, and a lubricant, wherein the water-soluble filler is mannitol and the glidant is colloidal anhydrous silica or a combination of silicon dioxide and magnesium aluminometasilicate.

**9.** The pharmaceutical composition according to any one of the preceding claims, wherein the water-soluble filler is D-mannitol, the water-insoluble filler is microcrystalline cellulose, the disintegrant is sodium starch glycolate, the glidant is colloidal anhydrous silica or a combination of colloidal anhydrous silica and magnesium aluminometasilicate, and the lubricant is magnesium stearate.

**10.** The pharmaceutical composition according to any one of the preceding claims, comprising:

- Bilastine polymorphic form 3 in an amount (wt% of the tablet) of 10-20%, preferably 12-18%, more preferably 16%;
- Mannitol in an amount of 40-70%, preferably 50-65%, more preferably 61%;
- Microcrystalline cellulose in an amount of 10-30%, preferably 12-20%, more preferably 14%;
- Sodium starch glycolate in an amount of 0.1-5%, preferably 2.5-4.5%, more preferably 4%;
- Colloidal anhydrous silica in an amount of 0.1-5%, preferably 2-4%, more preferably 2.8%; and
- Magnesium stearate in an amount of 0.1-5%, preferably 1-3%;

or

- Bilastine polymorphic form 3 in an amount (wt% of the tablet) of 10-20%;
- Mannitol in an amount of 40-70%;
- Microcrystalline cellulose in an amount of 10-30%;
- Sodium starch glycolate in an amount of 0.1-5%;
- Colloidal anhydrous silica in an amount of 0.1-5%;
- Magnesium aluminometasilicate in an amount of 0.1-5%; and
- Magnesium stearate in an amount of 0.1-5%;

or

- Bilastine polymorphic form 3 in an amount (wt% of the tablet) of 12-18%, more preferably 16%;
- Mannitol in an amount of 50-65%, more preferably 61%;
- Microcrystalline cellulose in an amount of 12-20%, more preferably 14%;
- Sodium starch glycolate in an amount of 2.5-4.5%, more preferably 4%;
- Colloidal anhydrous silica in an amount of 0.5-2%, more preferably 0.8%;
- Magnesium aluminometasilicate in an amount of 1-3%, more preferably 2%; and

- Magnesium stearate in an amount of 1-3%, more preferably 1.6%;
or
- Bilastine polymorphic form 3 in an amount (wt% of the tablet) of 12-18%, more preferably 16%;
- Mannitol in an amount of 50-65%, more preferably 58%;
- Microcrystalline cellulose in an amount of 15-25%, more preferably 20%;
- Sodium starch glycolate in an amount of 0.5-2%, more preferably 1.2%;
- Colloidal anhydrous silica in an amount of 0.5-2%, more preferably 0.8%;
- Magnesium aluminometasilicate in an amount of 1-3%, more preferably 2%; and
- Magnesium stearate in an amount of 1-3%, more preferably 1.6%.

**11.** The pharmaceutical composition according to any one of the preceding claims, wherein the tablet is a coated or uncoated immediate-release tablet, preferably uncoated.

**12.** The pharmaceutical composition according to any one of the preceding claims, wherein said composition is prepared by an essentially dry process without wet granulation.

**13.** The pharmaceutical composition according to any one of the preceding claims, wherein said composition is prepared by dry granulation of a blend of the components of at least claim 1 and optionally one or more of the additional components of any one or more of the preceding claims, and compression of the granules to a tablet, preferably wherein said composition is prepared by granulation of a blend of the components of claim 1 together with the additional components of any one or more of the preceding claims, roller compaction and granulation of the blend, milling the granules, lubricating the granules and subsequent compression of the granules to a tablet.

**14.** The pharmaceutical composition according to any one of claims 1 to 12, wherein said composition is prepared by direct compression of a blend of the components of claim 1 and optionally one or more of the additional components of any one or more of the preceding claims.

**15.** The pharmaceutical composition according to any one of the preceding claims for use as a medicament in the treatment of allergic rhino-conjunctivitis and/or urticaria.

**16.** Method of preparing a pharmaceutical composition in the form of a tablet according to any one of the preceding claims, wherein said composition is prepared by an essentially dry process without wet granulation.

**17.** Method of preparing a pharmaceutical composition in the form of a tablet, wherein said composition is prepared by dry granulation of a blend of the components of at least claim 1 and optionally one or more of the additional components of any one or more of the preceding claims, and compression of the granules to a tablet, preferably wherein said composition is prepared by granulation of a blend of the components of claim 1 together with the additional components of any one or more of the preceding claims, roller compaction and granulation of the blend, milling the granules, lubricating the granules and subsequent compression of the granules to a tablet.

**18.** Method of preparing a pharmaceutical composition in the form of a tablet, wherein said composition is prepared by direct compression of a blend of the components of claim 1 and optionally one or more of the additional components of any one or more of the preceding claims.

## Patentansprüche

**1.** Pharmazeutische Zusammensetzung in Form einer Tablette, umfassend a) kristalline Form 3 von Bilastin, wobei die kristalline Form 3 charakteristische Peaks bei 6,47, 12,81, 15,70 und 17,71 $\pm$ 0,2 Grad 2-Theta in einem Pulver-Röntgenbeugungsmuster aufweist, b) einen wasserlöslichen Füllstoff und gegebenenfalls c) einen wasserunlöslichen Füllstoff.

**2.** Pharmazeutische Zusammensetzung nach Anspruch 1, wobei die Zusammensetzung ferner ein Sprengmittel, ein Fließregulierungsmittel und/oder ein Schmiermittel umfasst.

**3.** Pharmazeutische Zusammensetzung nach einem der vorhergehenden Ansprüche, wobei der wasserlösliche Füllstoff ein Zucker ist, vorzugsweise Mannitol oder Laktose.

4. Pharmazeutische Zusammensetzung nach einem der vorhergehenden Ansprüche, wobei die Zusammensetzung einen wasserunlöslichen Füllstoff umfasst, der mikrokristalline Cellulose ist.

5. Pharmazeutische Zusammensetzung nach einem der vorhergehenden Ansprüche, wobei die Zusammensetzung ein Sprengmittel umfasst, ausgewählt aus Natriumstärkeglykolat, niedrigsubstituierter Hydroxypropylcellulose, vorgelatinierter Stärke oder Crospovidon, vorzugsweise Natriumstärkeglykolat oder Crospovidon.

6. Pharmazeutische Zusammensetzung nach einem der vorhergehenden Ansprüche, wobei die Zusammensetzung ein Fließregulierungsmittel umfasst, das Siliciumdioxid ist, vorzugsweise als kolloidales wasserfreies Siliciumdioxid, Magnesiumaluminometasilicat und/oder Talkum, wobei vorzugsweise das Fließregulierungsmittel eine Kombination von Siliciumdioxid und Magnesiumaluminometasilicat ist.

7. Pharmazeutische Zusammensetzung nach einem der vorhergehenden Ansprüche, wobei die Zusammensetzung ein Schmiermittel umfasst, das Magnesiumstearat ist.

8. Pharmazeutische Zusammensetzung nach einem der vorhergehenden Ansprüche, umfassend kristalline Form 3 von Bilastin, wobei die kristalline Form 3 charakteristische Peaks bei 6,47, 12,81, 15,70 und 17,71 $\pm$ 0,2 Grad 2-Theta in einem in einem Pulver-Röntgenbeugungsmuster aufweist, einen wasserlöslichen Füllstoff, einen wasserunlöslichen Füllstoff, ein Sprengmittel, ein Fließregulierungsmittel und ein Schmiermittel, wobei der wasserlösliche Füllstoff Mannitol und das Fließregulierungsmittel kolloidales wasserfreies Siliciumdioxid oder eine Kombination aus Siliciumdioxid und Magnesiumaluminometasilicat ist.

9. Pharmazeutische Zusammensetzung nach einem der vorhergehenden Ansprüche, wobei der wasserlösliche Füllstoff D-Mannitol, der wasserunlösliche Füllstoff mikrokristalline Cellulose, das Sprengmittel Natriumstärkeglykolat, das Fließregulierungsmittel kolloidales wasserfreies Siliciumdioxid oder eine Kombination aus kolloidalem wasserfreiem Siliciumdioxid und Magnesiumaluminometasilicat, und das Schmiermittel Magnesiumstearat ist.

10. Pharmazeutische Zusammensetzung nach einem der vorhergehenden Ansprüche, umfassend:

- Polymorphe Form 3 von Bilastin in einer Menge (Gew.-% der Tablette) von 10-20%, vorzugsweise 12-18%, bevorzugter 16%;
- Mannitol in einer Menge von 40-70%, vorzugsweise 50-65%, bevorzugter 61%;
- Mikrokristalline Cellulose in einer Menge von 10-30%, vorzugsweise 12-20%, bevorzugter 14%;
- Natriumstärkeglykolat in einer Menge von 0.1-5%, vorzugsweise 2.5-4.5%, bevorzugter 4%;
- Kolloidales wasserfreies Siliciumdioxid in einer Menge von 0.1-5%, vorzugsweise 2-4%, bevorzugter 2.8%; und
- Magnesiumstearat in einer Menge von 0.1-5%, vorzugsweise 1-3%;

oder

- Polymorphe Form 3 von Bilastin in einer Menge (Gew.-% der Tablette) von 10-20%;
- Mannitol in einer Menge von 40-70%;
- Mikrokristalline Cellulose in einer Menge von 10-30%;
- Natriumstärkeglykolat in einer Menge von 0.1-5%;
- Kolloidales wasserfreies Siliciumdioxid in einer Menge von 0.1-5%;
- Magnesiumaluminometasilicat in einer Menge von 0.1-5%; und
- Magnesiumstearat in einer Menge von 0.1-5%;

oder

- Polymorphe Form 3 von Bilastin in einer Menge (Gew.-% der Tablette) von 12-18%, bevorzugter 16%;
- Mannitol in einer Menge von 50-65%, bevorzugter 61%;
- Mikrokristalline Cellulose in einer Menge 12-20%, bevorzugter 14%;
- Natriumstärkeglykolat in einer Menge von 2.5-4.5%, bevorzugter 4%;
- Kolloidales wasserfreies Siliciumdioxid in einer Menge von 0.5-2%, bevorzugter 0.8%;
- Magnesiumaluminometasilicat in einer Menge von 1-3%, bevorzugter 2%; und
- Magnesiumstearat in einer Menge von 1-3%, bevorzugter 1.6%;

oder

- Polymorphe Form 3 von Bilastin in einer Menge (Gew.-% der Tablette) von 12-18%, bevorzugter 16%;
- Mannitol in einer Menge von 50-65%, bevorzugter 58%;
- Mikrokristalline Cellulose in einer Menge von 15-25%, bevorzugter 20%;
- Natriumstärkeglykolat in einer Menge von 0.5-2%, bevorzugter 1.2%;
- Kolloidales wasserfreies Siliciumdioxid in einer Menge von 0.5-2%, bevorzugter 0.8%;

- Magnesiumaluminometasilicat in einer Menge von 1-3%, bevorzugter 2%; und
- Magnesiumstearat in einer Menge von 1-3%, bevorzugter 1.6%.

**11.** Pharmazeutische Zusammensetzung nach einem der vorhergehenden Ansprüche, wobei die Tablette eine beschichtete oder unbeschichtete Tablette mit sofortiger Freisetzung ist, vorzugsweise eine unbeschichtete.

**12.** Pharmazeutische Zusammensetzung nach einem der vorhergehenden Ansprüche, wobei die Zusammensetzung durch ein im Wesentlichen trockenes Verfahren ohne Nassgranulierung hergestellt wird.

**13.** Pharmazeutische Zusammensetzung nach einem der vorhergehenden Ansprüche, wobei die Zusammensetzung durch Trockengranulierung einer Mischung der Komponenten nach mindestens Anspruch 1 und gegebenenfalls einer oder mehrerer der zusätzlichen Komponenten nach einem oder mehreren der vorhergehenden Ansprüche und Verpressen des Granulats zu einer Tablette hergestellt wird, wobei vorzugsweise die Zusammensetzung durch Granulierung einer Mischung der Komponenten nach Anspruch 1 zusammen mit den zusätzlichen Komponenten nach einem oder mehreren der vorhergehenden Ansprüche, Walzenkompaktierung und Granulierung der Mischung, Mahlen des Granulats, Schmieren des Granulats und anschließendes Verpressen des Granulats zu einer Tablette hergestellt wird.

**14.** Pharmazeutische Zusammensetzung nach einem der Ansprüche 1 bis 12, wobei die Zusammensetzung durch Direktverpressung einer Mischung der Komponenten nach Anspruch 1 und gegebenenfalls einer oder mehrerer der zusätzlichen Komponenten nach einem oder mehreren der vorhergehenden Ansprüche hergestellt wird.

**15.** Pharmazeutische Zusammensetzung nach einem der vorhergehenden Ansprüche zur Verwendung als Medikament zur Behandlung von allergischer Rhino-Konjunktivitis und/oder Urtikaria.

**16.** Verfahren zur Herstellung einer pharmazeutischen Zusammensetzung in Form einer Tablette nach einem der vorhergehenden Ansprüche, wobei die Zusammensetzung durch ein, im Wesentlichen trockenes, Verfahren ohne Nassgranulierung hergestellt wird.

**17.** Verfahren zur Herstellung einer pharmazeutischen Zusammensetzung in Form einer Tablette, wobei die Zusammensetzung durch Trockengranulierung einer Mischung der Komponenten nach mindestens Anspruch 1 und gegebenenfalls einer oder mehrerer der zusätzlichen Komponenten nach einem oder mehreren der vorhergehenden Ansprüche und Verpressen des Granulats zu einer Tablette hergestellt wird, wobei vorzugsweise die Zusammensetzung durch Granulierung einer Mischung der Komponenten von Anspruch 1 zusammen mit den zusätzlichen Komponenten nach einem oder mehreren der vorhergehenden Ansprüche, Walzenkompaktierung und Granulierung der Mischung, Mahlen des Granulats, Schmieren des Granulats und anschließendes Verpressen des Granulats zu einer Tablette hergestellt wird.

**18.** Verfahren zur Herstellung einer pharmazeutischen Zusammensetzung in Form einer Tablette, wobei die besagte Zusammensetzung durch Direktverpressung einer Mischung der Komponenten nach Anspruch 1 und gegebenenfalls einer oder mehrerer der zusätzlichen Komponenten nach einem oder mehreren der vorhergehenden Ansprüche hergestellt wird.

## Revendications

**1.** Composition pharmaceutique sous forme de comprimé, comprenant a) une forme cristalline 3 de bilastine, dans laquelle la forme cristalline 3 a des pics caractéristiques à 6,47, 12,81, 15,70 et 17,71 ± 0,2 degrés 2-thêta dans un diagramme de diffraction des rayons X sur poudre, b) une charge soluble dans l'eau, et éventuellement c) une charge insoluble dans l'eau.

**2.** Composition pharmaceutique selon la revendication 1, dans laquelle la composition comprend en outre un désintégrant, un agent de glissement et/ou un lubrifiant.

**3.** Composition pharmaceutique selon l'une quelconque des revendications précédentes, dans laquelle la charge soluble dans l'eau est un sucre, de préférence le mannitol ou le lactose.

**4.** Composition pharmaceutique selon l'une quelconque des revendications précédentes, dans laquelle la composition

comprend une charge insoluble dans l'eau, qui est de la cellulose microcristalline.

5. Composition pharmaceutique selon l'une quelconque des revendications précédentes, dans laquelle la composition comprend un désintégrant, choisi parmi le glycolate d'amidon sodique, l'hydroxypropylcellulose faiblement substituée, l'amidon prégélatinisé ou la crospovidone, de préférence le glycolate d'amidon sodique ou la crospovidone.

6. Composition pharmaceutique selon l'une quelconque des revendications précédentes, dans laquelle la composition comprend un agent de glissement, qui est du dioxyde de silicium, de préférence sous forme de silice colloïdale anhydre, d'aluminométasilicate de magnésium et/ou de talc, de préférence dans laquelle l'agent de glissement est une combinaison de dioxyde de silicium et d'aluminométasilicate de magnésium.

7. Composition pharmaceutique selon l'une quelconque des revendications précédentes, dans laquelle la composition comprend un lubrifiant, qui est du stéarate de magnésium.

8. Composition pharmaceutique selon l'une quelconque des revendications précédentes, comprenant une forme cristalline 3 de bilastine, dans laquelle la forme cristalline 3 a des pics caractéristiques à 6,47, 12,81, 15,70 et 17,71 ± 0,2 degrés 2-thêta dans un diagramme de diffraction des rayons X sur poudre, une charge soluble dans l'eau, une charge insoluble dans l'eau, un désintégrant, un agent de glissement et un lubrifiant, dans laquelle la charge soluble dans l'eau est le mannitol et l'agent de glissement est de la silice colloïdale anhydre ou une combinaison de dioxyde de silicium et d'aluminométasilicate de magnésium.

9. Composition pharmaceutique selon l'une quelconque des revendications précédentes, dans laquelle la charge soluble dans l'eau est le D-mannitol, la charge insoluble dans l'eau est la cellulose microcristalline, le désintégrant est le glycolate d'amidon sodique, le glissant est de la silice colloïdale anhydre ou une combinaison de silice colloïdale anhydre et d'aluminométasilicate de magnésium, et le lubrifiant est le stéarate de magnésium.

10. Composition pharmaceutique selon l'une quelconque des revendications précédentes, comprenant:

- la forme polymorphe 3 de bilastine en une quantité (% en poids du comprimé) de 10 à 20% , préférablement de 12 à 18% , plus préférablement de 16% ;
- le mannitol en une quantité de 40 à 70% , préférablement de 50 à 65% , plus préférablement de 61%;
- la cellulose microcristalline en une quantité de 10 à 30% préférablement de 12 à 20% plus préférablement de 14% ;
- le glycolate d'amidon sodique en une quantité de 0,1 à 5% , préférablement de 2,5 à 4,5% , plus préférablement de 4% ;
- la silice colloïdale anhydre en une quantité de 0,1 à 5% , préférablement de 2 à 4% , plus préférablement de 2,8%;
- le stéarate de magnésium en une quantité de 0,1 à 5% préférablement de 1 à 3% ;

ou

- la forme polymorphe 3 de bilastine en une quantité (% en poids du comprimé) de 10 à 20% ;
- le mannitol en une quantité de 40 à 70% ;
- la cellulose microcristalline en une quantité de 10 à 30% ;
- le glycolate d'amidon sodique en une quantité de 0,1 à 5% ;
- la silice colloïdale anhydre en une quantité de 0,1 à 5% ;
- l'aluminométasilicate de magnésium en une quantité de 0,1 à 5% ; et
- le stéarate de magnésium en une quantité de 0,1 à 5% ;

ou

- la forme polymorphe 3 de bilastine en une quantité (% en poids du comprimé) de 12 à 18% , plus préférablement de 16% ;
- le mannitol en une quantité de 50 à 65% , plus préférablement de 61% ;
- la cellulose microcristalline en une quantité de 12 à 20% , plus préférablement de 14% ;
- le glycolate d'amidon sodique en une quantité de 2,5 à 4,5% , plus préférablement de 4% ;
- la silice colloïdale anhydre en une quantité de 0,5 à 2% , plus préférablement de 0,8% ;
- l'aluminométasilicate de magnésium en une quantité de 1 à 3% , plus préférablement de 2% ; et
- le stéarate de magnésium en une quantité de 1 à 3% , plus préférablement de 1,6% ;

ou

- la forme polymorphe 3 de bilastine en une quantité (% en poids du comprimé) de 12 à 18% , plus préférablement de 16% ;

- le mannitol en une quantité de 50 à 65% , plus préférablement de 58% ;
- la cellulose microcristalline en une quantité de 15 à 25% , plus préférablement de 20% ;
- le glycolate d'amidon sodique en une quantité de 0,5 à 2% , plus préférablement de 1,2% ;
- la silice colloïdale anhydre en une quantité de 0,5 à 2% , plus préférablement de 0,8% ;
- l'aluminométasilicate de magnésium en une quantité de 1 à 3% , plus préférablement de 2% ; et
- le stéarate de magnésium en une quantité de 1 à 3% , plus préférablement de 1,6%.

**11.** Composition pharmaceutique selon l'une quelconque des revendications précédentes, dans laquelle le comprimé est un comprimé à libération immédiate enrobé ou non enrobé, de préférence non enrobé.

**12.** Composition pharmaceutique selon l'une quelconque des revendications précédentes, dans laquelle ladite composition est préparée par un procédé essentiellement sec sans granulation humide.

**13.** Composition pharmaceutique selon l'une quelconque des revendications précédentes, dans laquelle ladite composition est préparée par granulation à sec d'un mélange des composants selon au moins la revendication 1 et éventuellement de l'un ou de plusieurs des composants supplémentaires selon l'une quelconque ou plusieurs des revendications précédentes, et compression des granules en un comprimé, de préférence dans laquelle ladite composition est préparée par granulation d'un mélange des composants selon la revendication 1 conjointement avec les composants supplémentaires selon l'une quelconque ou plusieurs des revendications précédentes, compactage au rouleau et granulation du mélange, broyage des granules, lubrification des granules et compression subséquente des granules en un comprimé.

**14.** Composition pharmaceutique selon l'une quelconque des revendications 1 à 12, dans laquelle ladite composition est préparée par compression directe d'un mélange des composants selon la revendication 1 et éventuellement de l'un ou de plusieurs des composants supplémentaires selon l'une quelconque ou plusieurs des revendications précédentes.

**15.** Composition pharmaceutique selon l'une quelconque des revendications précédentes pour une utilisation comme médicament dans le traitement de la rhino-conjonctivite allergique et/ou de l'urticaire.

**16.** Procédé de préparation d'une composition pharmaceutique sous la forme d'un comprimé selon l'une quelconque des revendications précédentes, dans laquelle ladite composition est préparée par un procédé essentiellement sec sans granulation humide.

**17.** Procédé de préparation d'une composition pharmaceutique sous la forme d'un comprimé, dans laquelle ladite composition est préparée par granulation à sec d'un mélange des composants selon au moins la revendication 1 et éventuellement de l'un ou de plusieurs des composants supplémentaires selon l'une quelconque ou plusieurs des revendications précédentes, et compression des granules en un comprimé, de préférence dans laquelle ladite composition est préparée par granulation d'un mélange des composants selon la revendication 1 conjointement avec les composants supplémentaires selon l'une quelconque ou plusieurs des revendications précédentes, compactage au rouleau et granulation du mélange, broyage des granules, lubrification des granules et compression subséquente des granules en un comprimé.

**18.** Procédé de préparation d'une composition pharmaceutique sous la forme d'un comprimé, dans laquelle ladite composition est préparée par compression directe d'un mélange des composants selon la revendication 1 et éventuellement de l'un ou de plusieurs des composants supplémentaires selon l'une quelconque ou plusieurs des revendications précédentes.

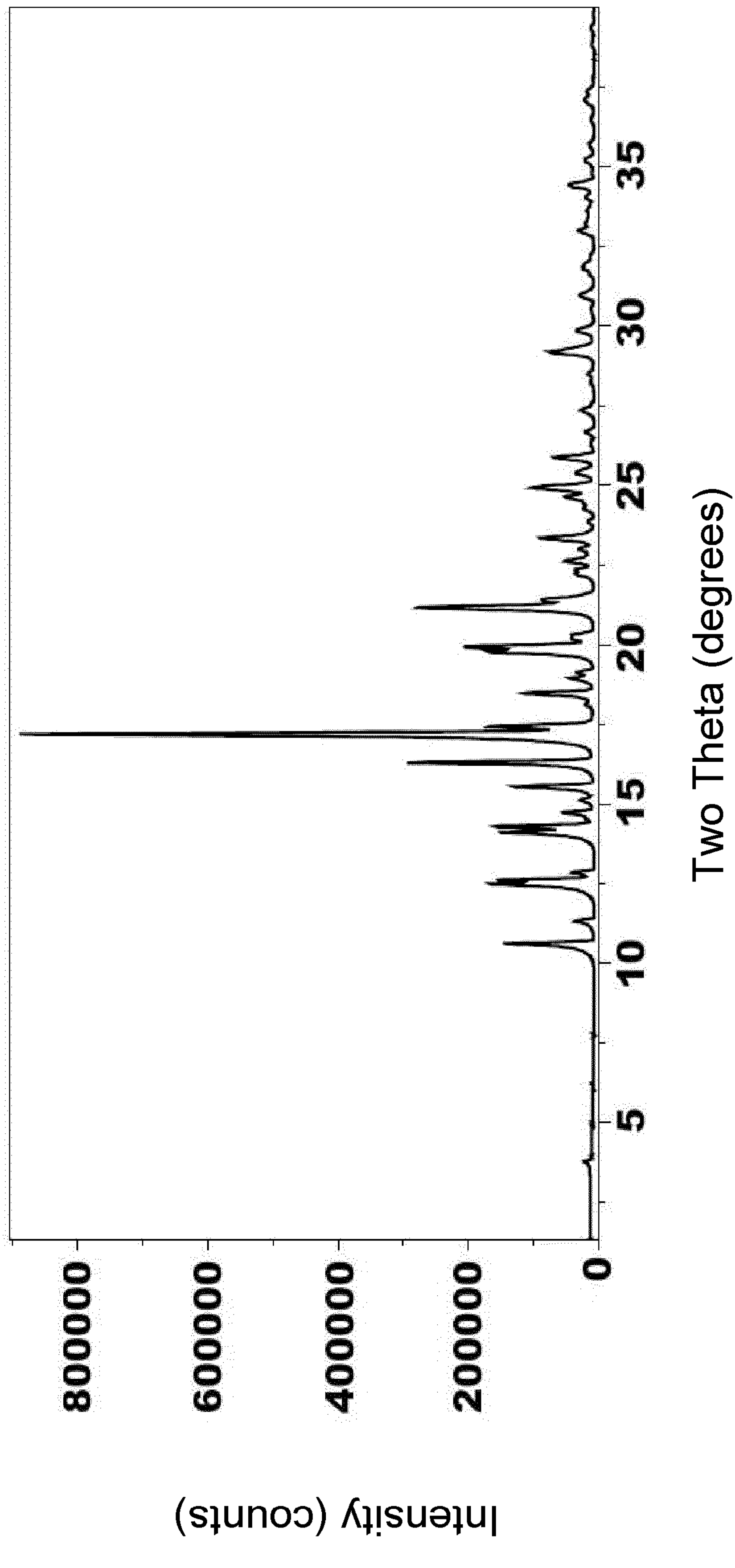
Intensity (counts)
8000000
6000000
4000000
2000000
0
5
10
15
20
25
30
35
Two Theta (degrees)

FIG. 1

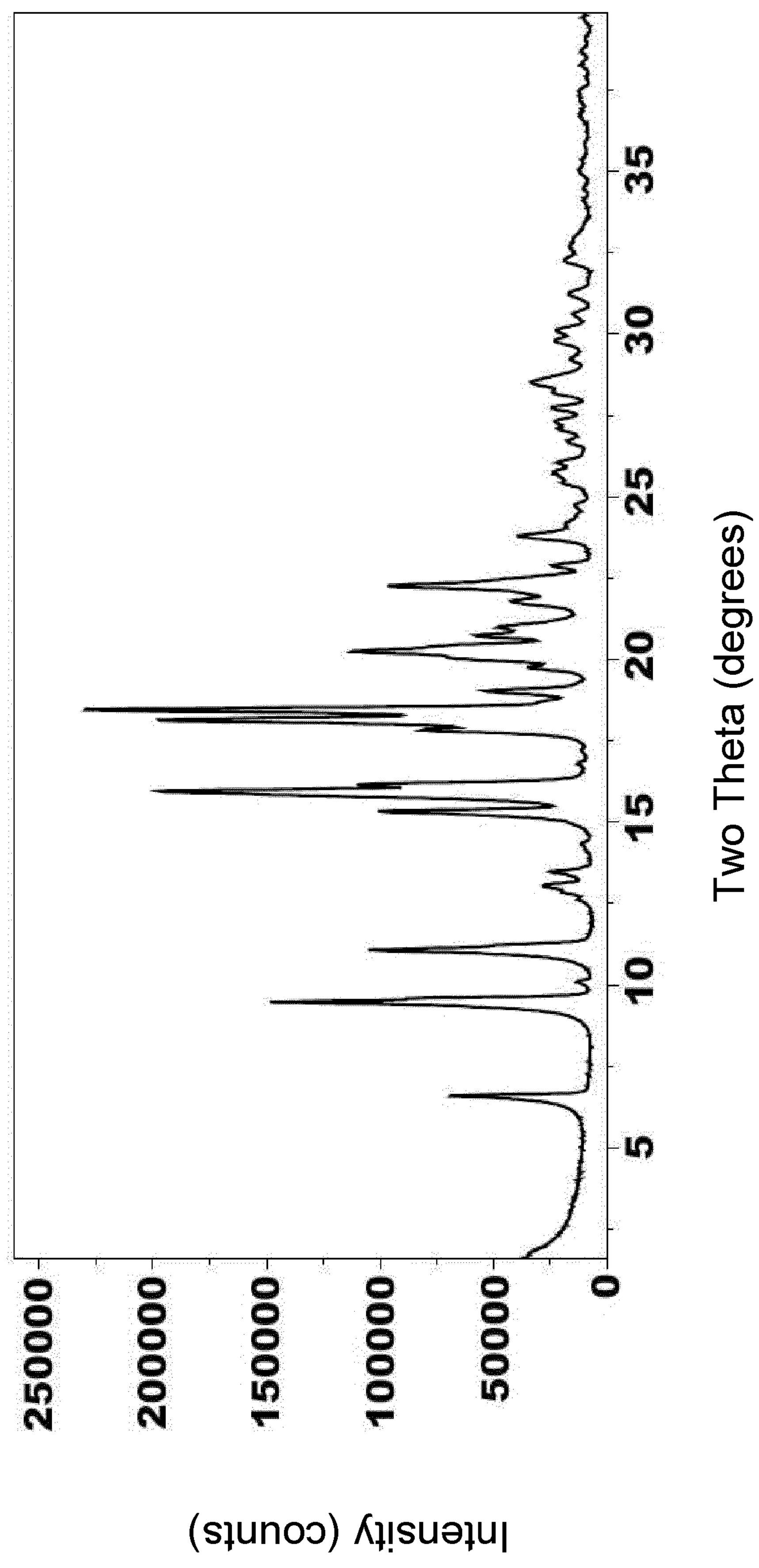
Intensity (counts)
0
50000
100000
150000
200000
250000
5
10
15
20
25
30
35
Two Theta (degrees)

FIG. 2

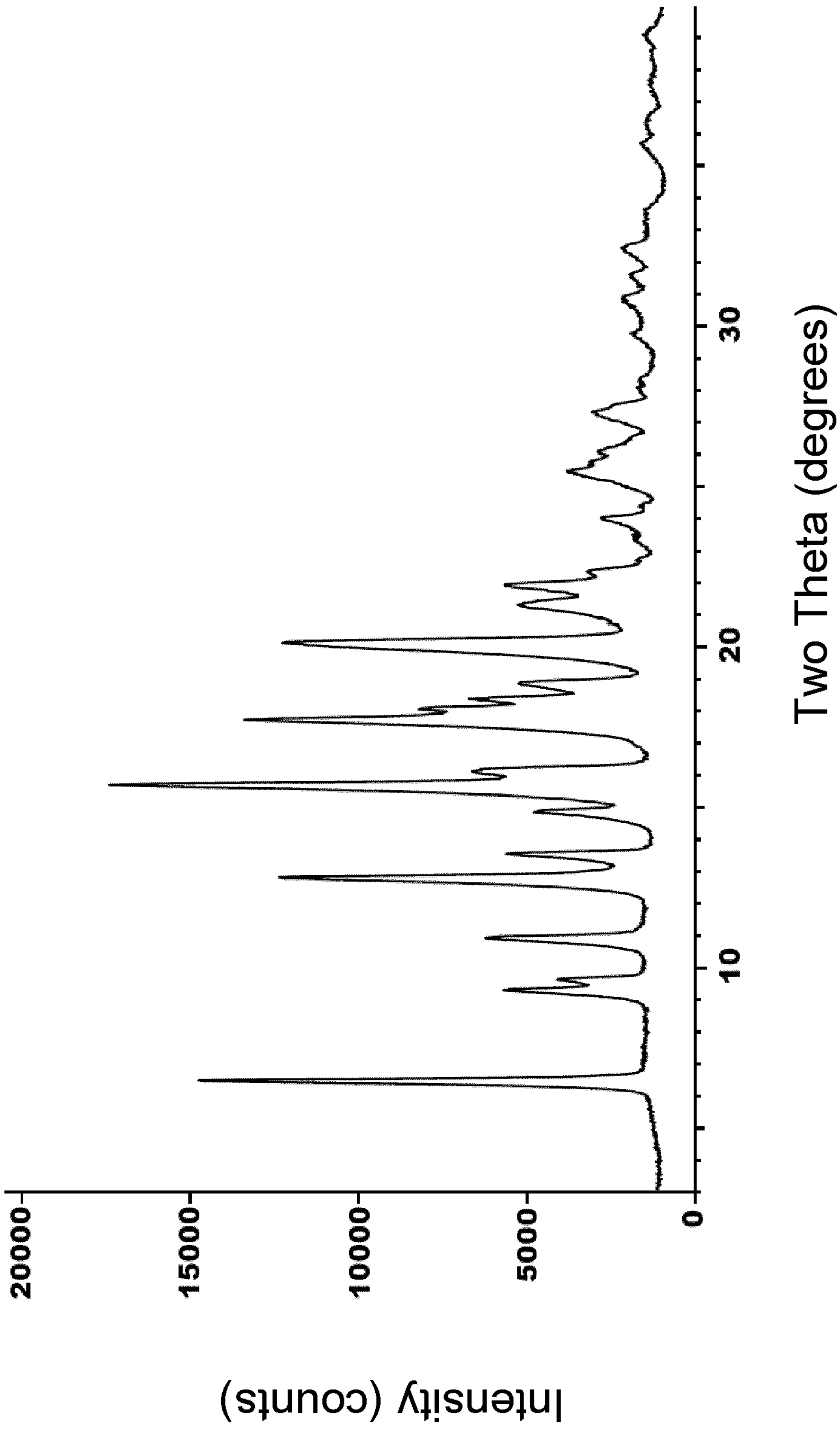
Intensity (counts)
20000
15000
10000
5000
0
10
20
30
Two Theta (degrees)

FIG. 3

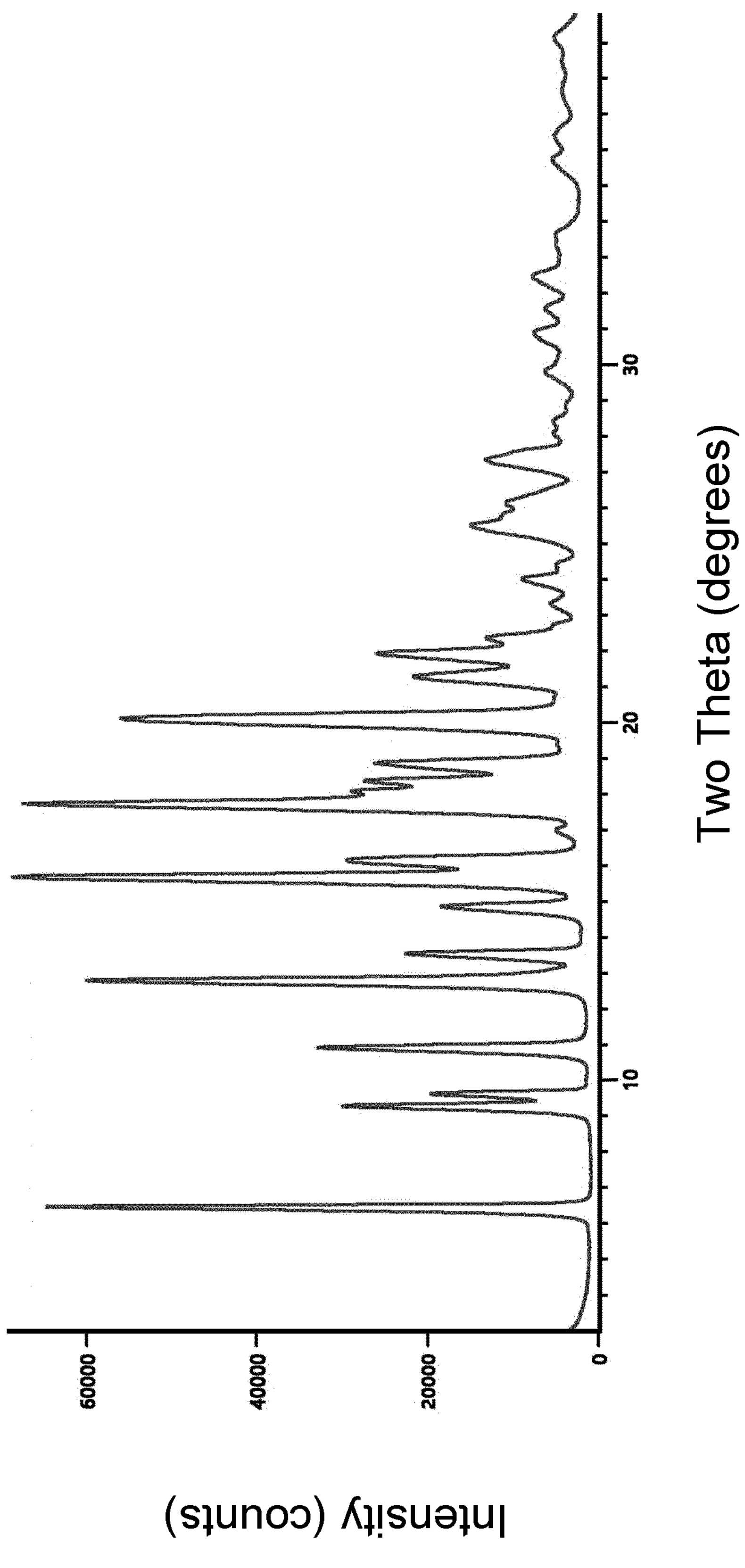
Intensity (counts)
60000
40000
20000
0
10
20
30
Two Theta (degrees)

FIG. 4

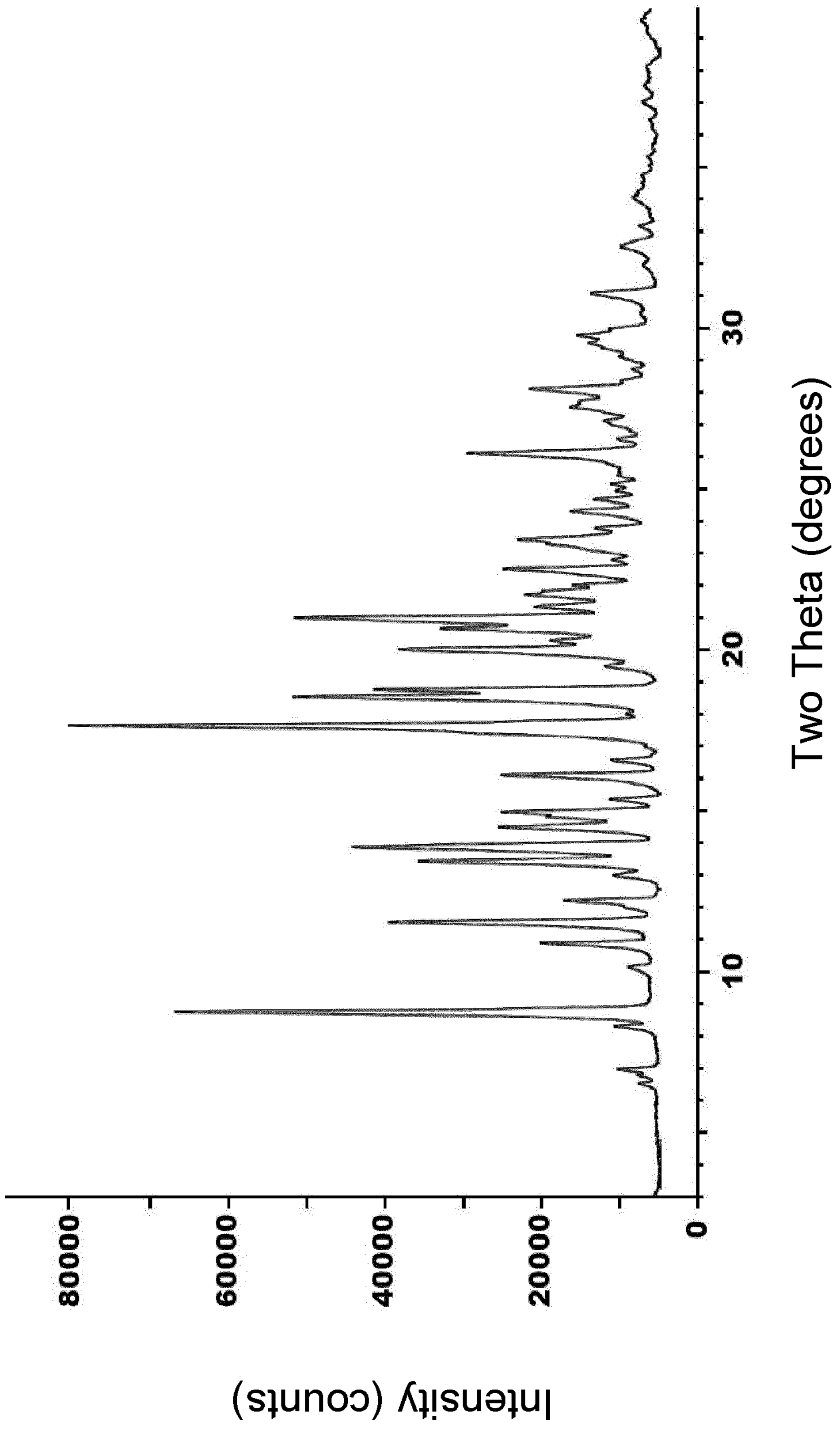
Intensity (counts)
80000
60000
40000
20000
0
10
20
30
Two Theta (degrees)

FIG. 5

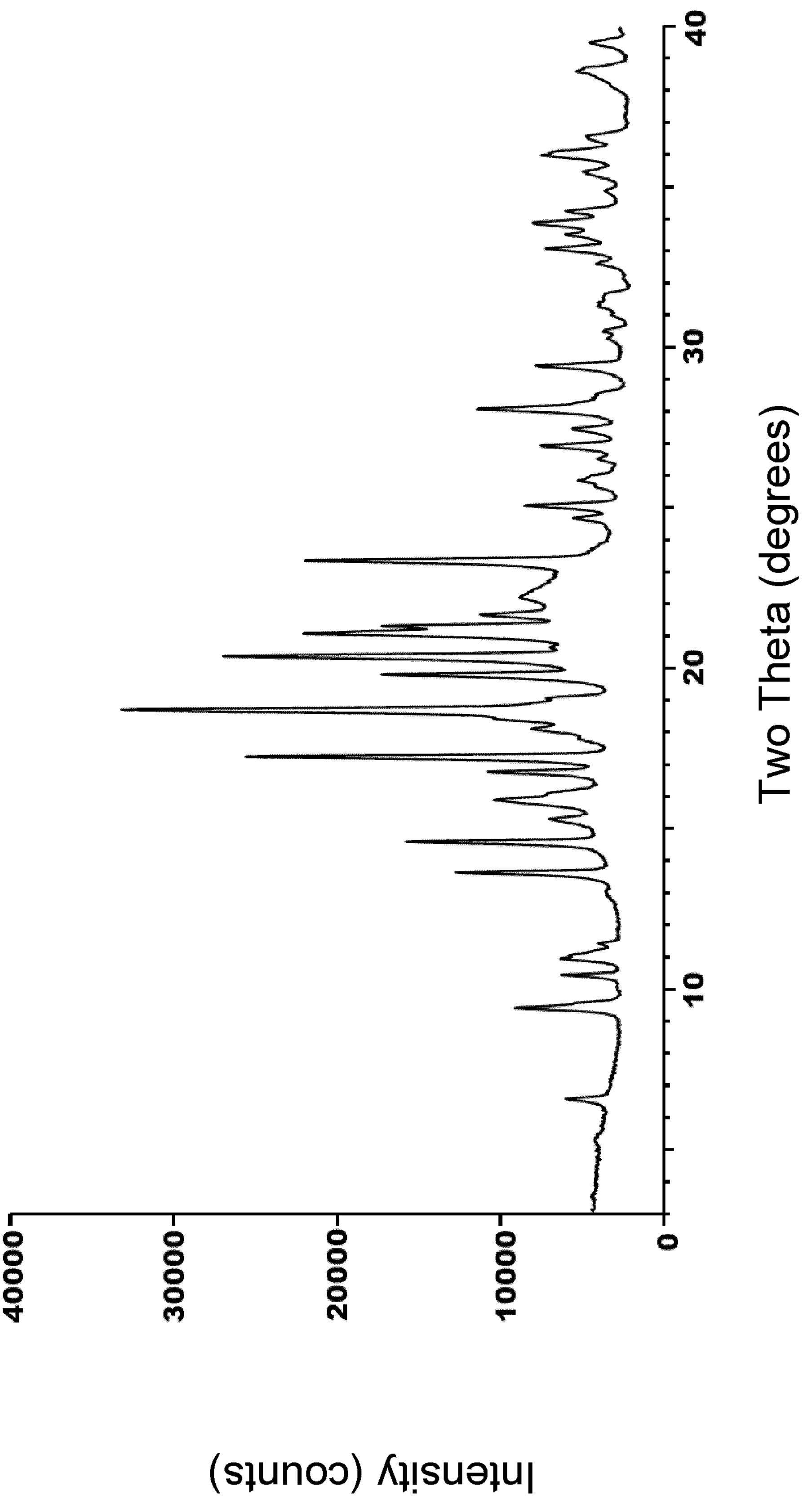
Intensity (counts)
40000
30000
20000
10000
0
10
20
30
40
Two Theta (degrees)

FIG. 6

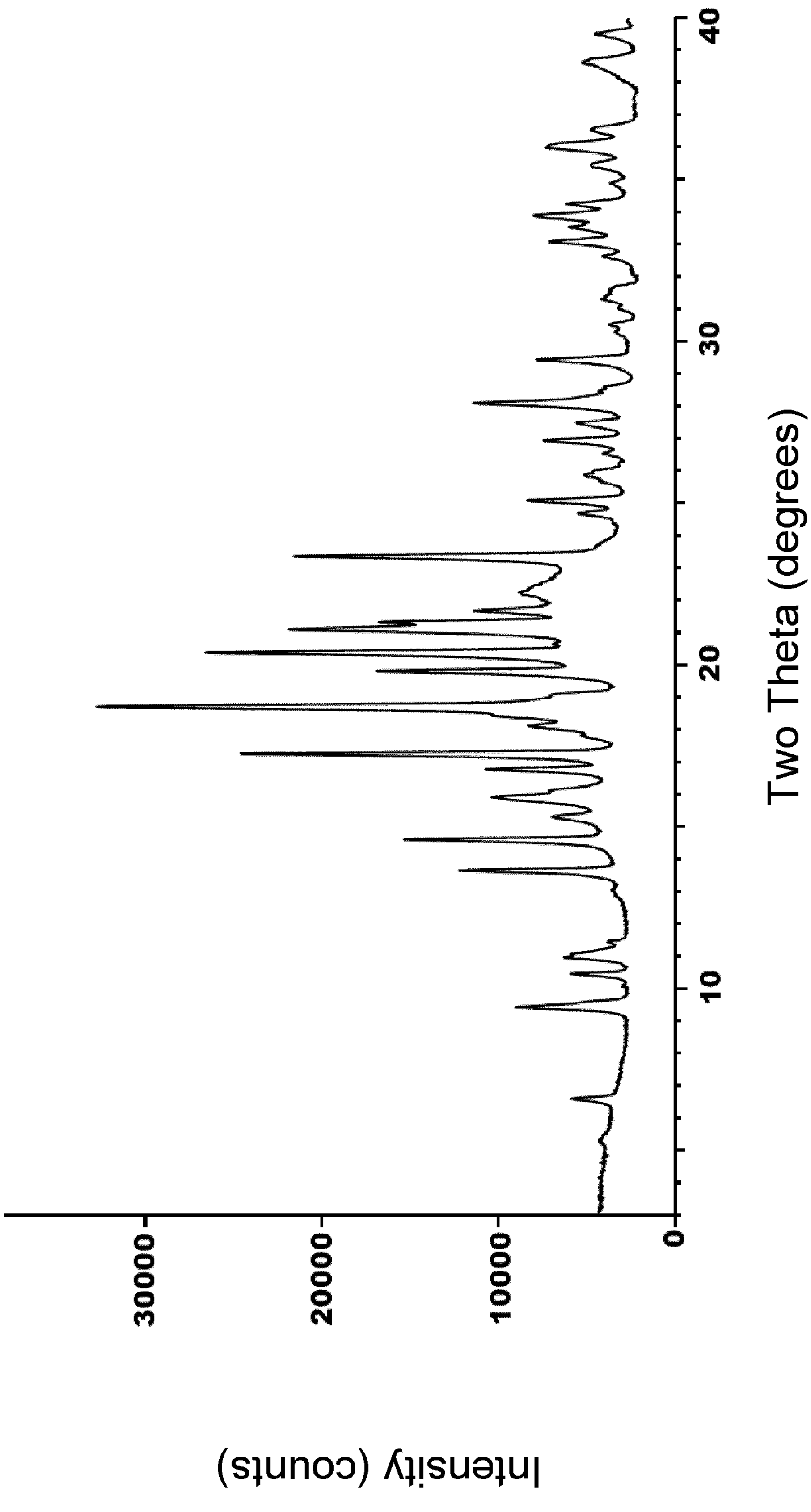
Intensity (counts)
0
10000
20000
30000
10
20
30
40
Two Theta (degrees)

FIG. 7

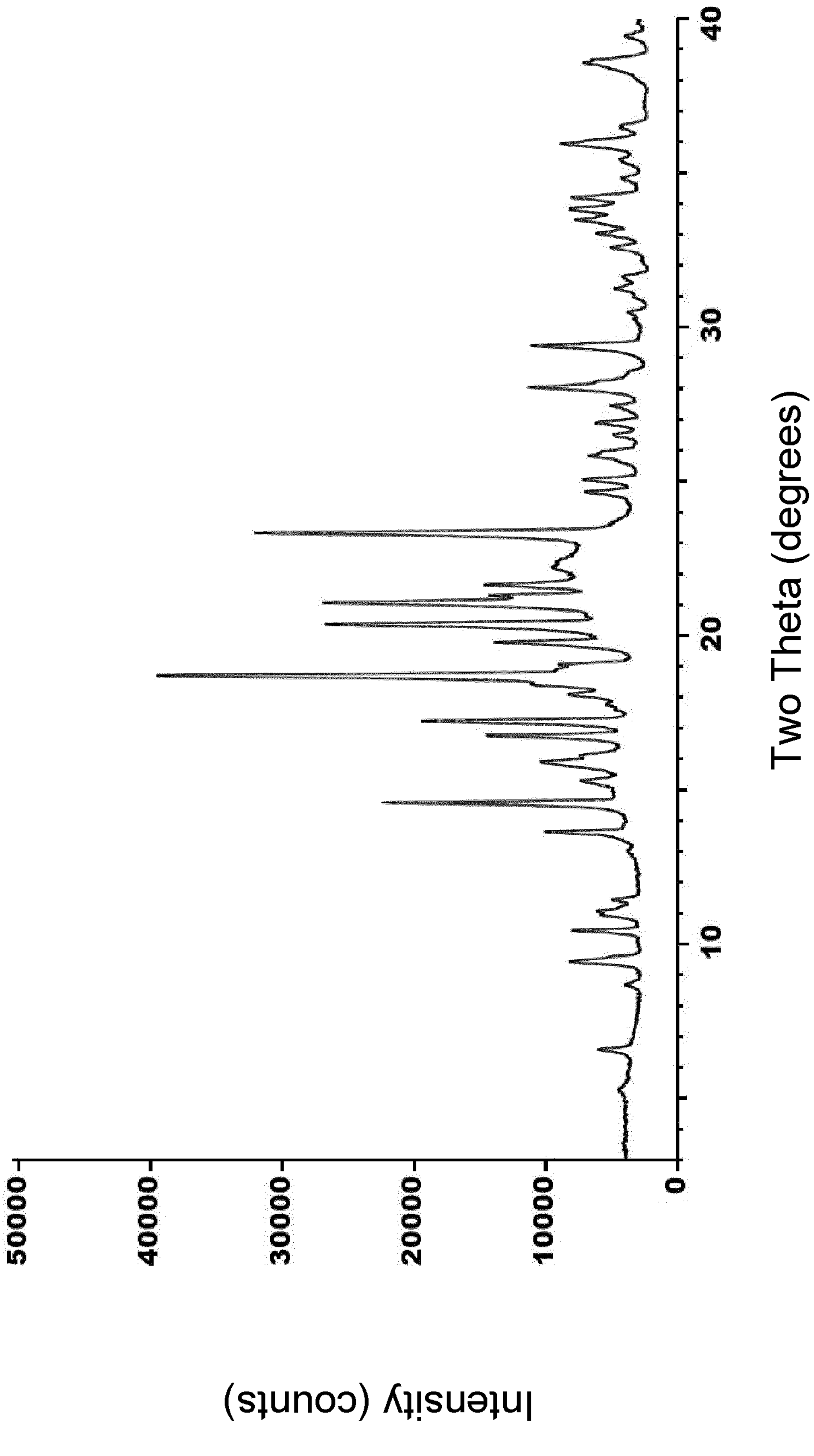
Intensity (counts)
0
10000
20000
30000
40000
50000
10
20
30
40
Two Theta (degrees)

FIG. 8

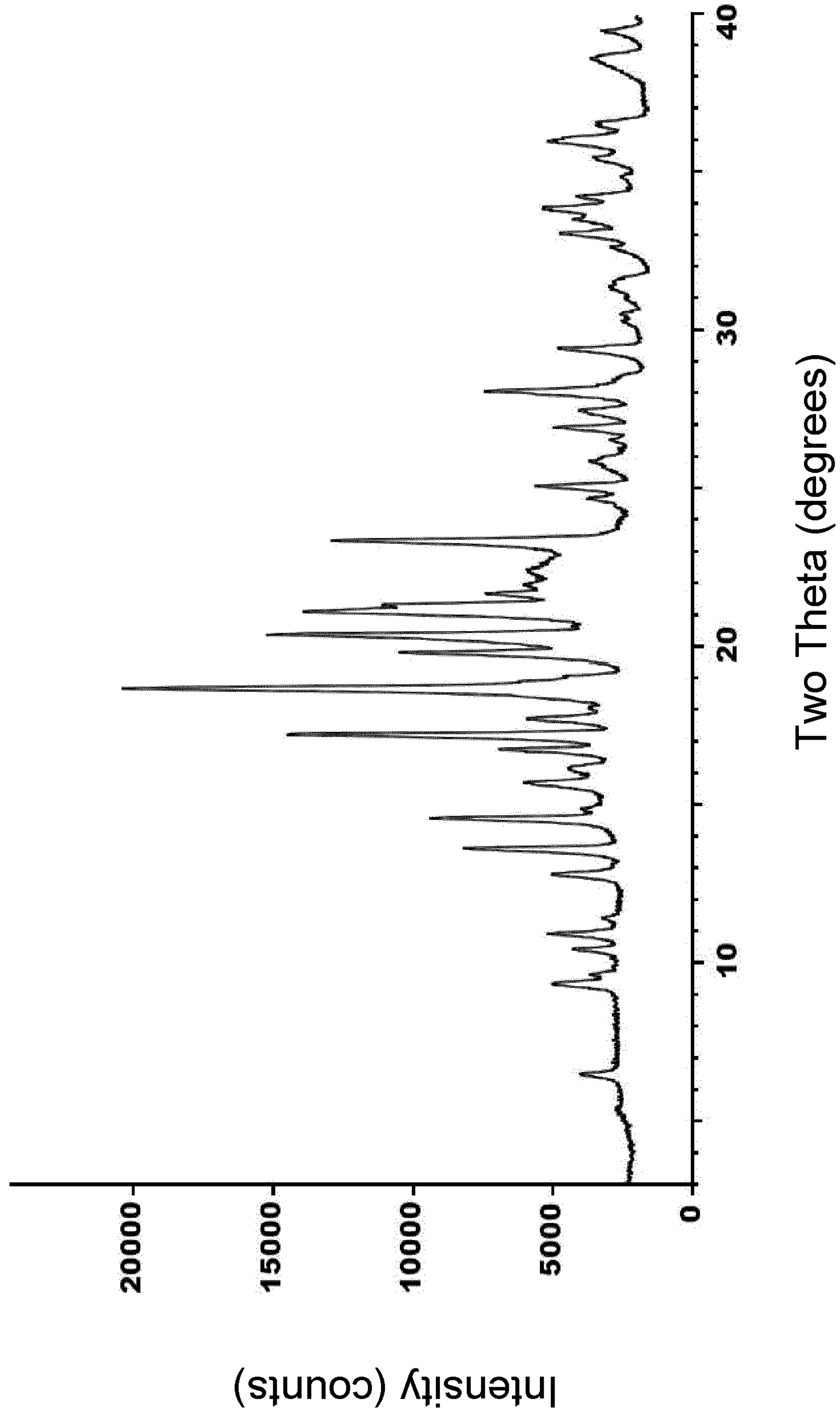
Intensity (counts)
0
5000
10000
15000
20000
10
20
30
40
Two Theta (degrees)

FIG. 9

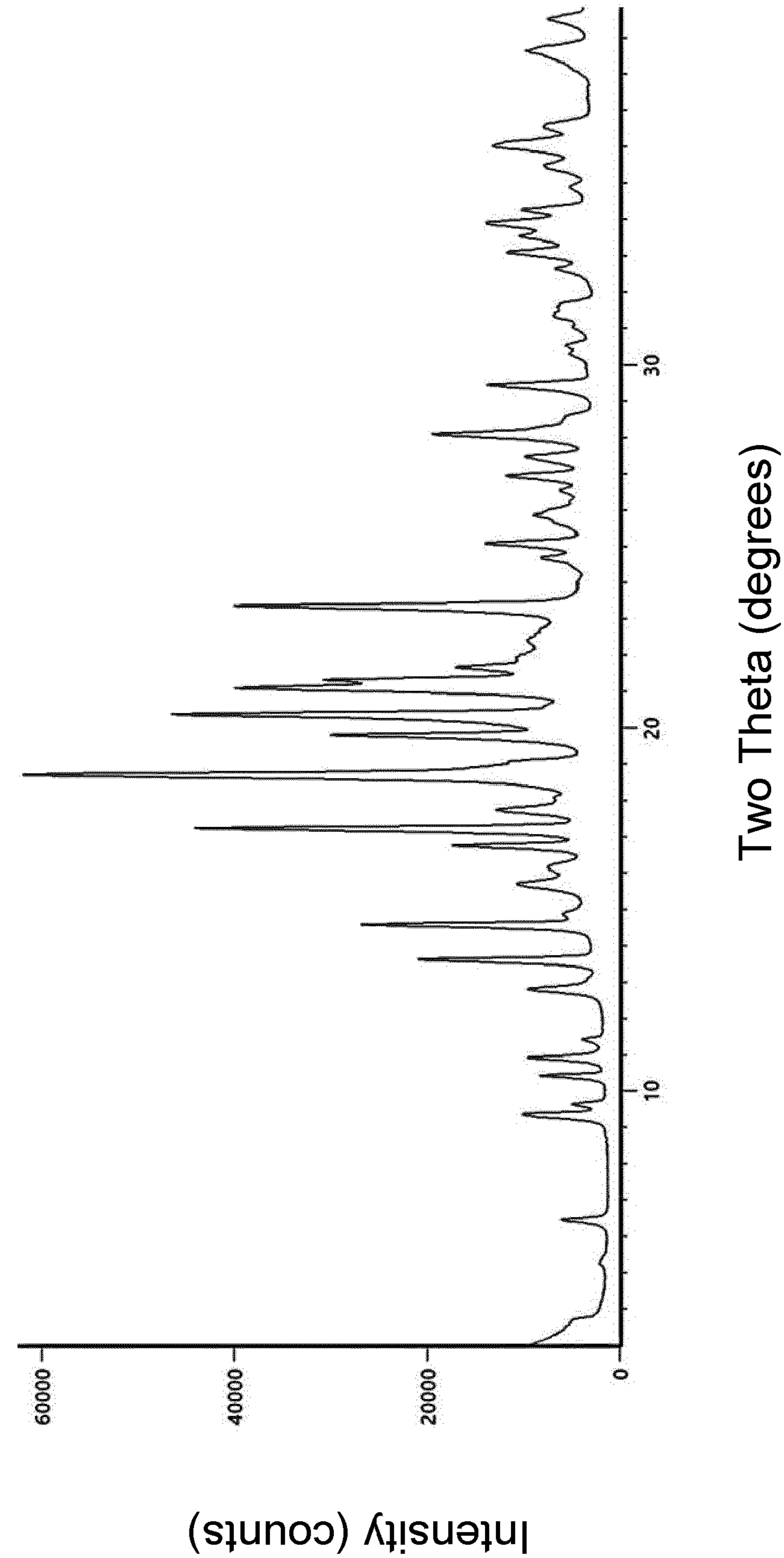
60000
40000
20000
0
10
20
30
Two Theta (degrees)
Intensity (counts)

FIG. 10

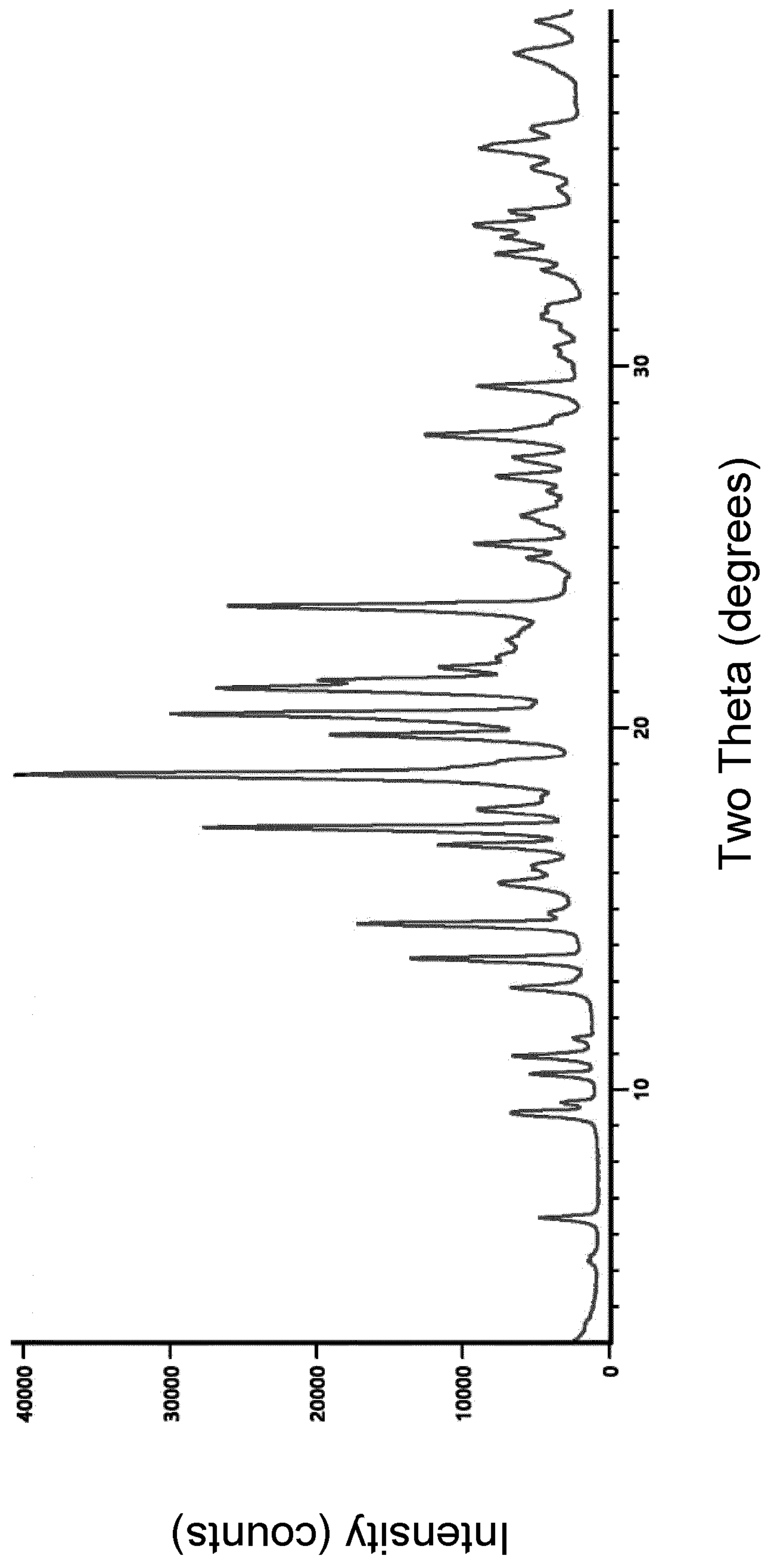
Intensity (counts)
0
10000
20000
30000
40000
10
20
30
Two Theta (degrees)

FIG. 11

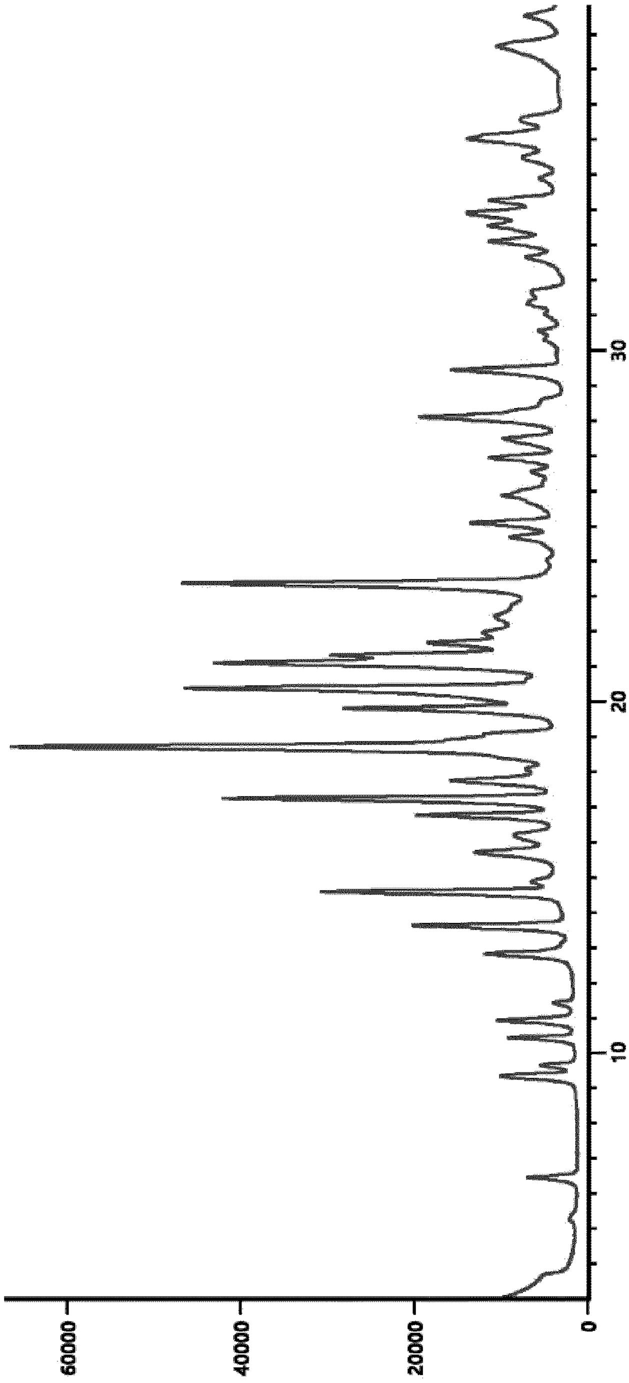
Intensity (counts)
60000
40000
20000
0
10
20
30
Two Theta (degrees)

FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0818454 A
- EP 1505066 A
- EP 818454 A
- WO 2014026657 A
- SK 7066 Y1
- WO 2017017301 A
- WO 2017167949 A
- CN 106692090A

### Non-patent literature cited in the description

- *CHEMICAL ABSTRACTS,* 202189-78-4
- *CHEMICAL ABSTRACTS,* 12511-31-8
- **WATERMAN KC.** The application of the Accelerated Stability Assessment Program (ASAP) to quality by design (QbD) for drug oroduct stability. *AAPS PharmSciTech,* September 2011, vol. 12 (3